(19) Europäisches Patentamt

European Patent Office

Office européen des brevets

(11) Publication number: **0 277 792**
**A2**

# (12) EUROPEAN PATENT APPLICATION

(21) Application number: **88300824.5**

(22) Date of filing: **01.02.88**

(51) Int. Cl.⁴: **A 61 B 1/04**

(30) Priority: 31.01.87 JP 21459/87
31.01.87 JP 21461/87
10.03.87 JP 54599/87
17.03.87 JP 61688/87

(43) Date of publication of application:
**10.08.88** Bulletin **88/32**

(84) Designated Contracting States:
**AT BE CH DE ES FR GB GR IT LI LU NL SE**

(71) Applicant: **OLYMPUS OPTICAL CO., LTD.**
43-2, 2-chome, Hatagaya Shibuya-ku
**Tokyo 151 (JP)**

(72) Inventor: **Hibino, Hiroki**
Mezondonoabamu Hachioji 413, 560-11, Kitano-machi
Hachioji-shi Tokyo (JP)

**Kimura, Kenji**
2-28-13, Nishisuna-cho
Tachikawa-shi Tokyo (JP)

**Kanno, Masahide**
Utsugidae 1-2-401, 1067, Utsugi-cho
Hachioji-shi Tokyo (JP)

**Nishikori, Toshiaki**
Pakuhaitsu 704, 5-4-4, Sagamihara
Sagamihara-shi Kanagawa-ken (JP)

**Yoshinaga, Jun**
3-25-10, Shinmei
Hino-shi Tokyo (JP)

**Kidawara, Atsushi**
6-25-7, Nishiki-cho
Tachikawa-shi Tokyo (JP)

**Yabe, Hisao**
4-1-19, Myojin-cho
Tachikawa-shi Tokyo (JP)

**Kato, Shinichi**
3-84-16, Katsunuma
Oume-shi Tokyo (JP)

**Takamura, Koji**
Kopoyasui 202, 538-3, Kitano-machi
Hachioji-shi Tokyo (JP)

**Nakamura, Takeaki**
6-9-8, Asahigaoka
Hino-shi Tokyo (JP)

(74) Representative: **Kennedy, Victor Kenneth et al**
G.F. REDFERN & CO. Marlborough Lodge 14 Farncombe
Road
Worthing West Sussex BN11 2BT (GB)

(54) **Endoscope imaging systems.**

(57) An endoscope imaging system comprising a frame sequential type colour imaging device; a colour imaging device provided with a colour filter; an illuminting light output source which can selectively output illuminating lights having the characteristic required to correspond to these different colour imaging devices; a signal processing device for processing the signals corresponding to the respecticve colour imaging devices; and a colour monitor displaying the processed colour video signals so that any said colour imaging device can be used.

EP 0 277 792 A2

FIG.1

**Description**

ENDOSCOPE IMAGING SYSTEMS:

The invention relates to endoscope imaging systems which can be used with endoscopes having different colour imaging systems.

Recently, instead of endoscopes having an optical viewing system, for example an optical-fibre scope, wherein an optical image formed by an objective in the tip of an insertable sheath portion is transmitted to a base unit via an image guide formed of an optical-fiber bunch, there has been a change to more practically useful electronic endoscopes (which shall hereinafter be called electronic endoscopes or electronic scopes), wherein an optical image formed by an objective on a photo-electrid transducer is converted to an electric signal, transmitted to the base unit, and can then be fed through a video processor to be displayed on a colour monitor.

The electronic scopes now used for upper and lower digestive tubes generally have an insertion sheath diameter of 10 mm, the diameter being required to provide a display of the necessary resolution. However, for example, an endoscope for the bronchus is usually required to have a diameter less than 5 mm and, in order to produce an electronic scope having an insertion sheath with the thin diameter needed for the bronchus, an imaging device having a reduced resolution, and consequently a smaller number of pixels per image, has to be used.

Where the number of pixels has to be small, then, in order to prevent too drastic a reduction of the resolution, a field sequential type colour imaging system can be used, wherein an object is illuminated sequentially with lights of respective wavelengths, e.g., red, blue and green, and is displayed field sequentially, each respective image produced from the illumination radiated at any instant having three times the otherwise available resolutions, so that the images displayed combine to give an advantageous colour display compared with that of a colour display system wherein a single colour mosaic filter is used to produce displayed images of full colour each time.

Where the number of pixels per frame can be increased, because of thicker diameter is available and a sufficient resolution can be obtained, a mosaic type colour imaging system using a mosaic filter can then be adopted equally effectively without problems.

In the case of using such an electronic scope, a video processor wherein a signal is produced to be a video signal which can be displayed by a colour monitor is used in addition to the light source normally used in a filter scope.

Prior art constructions have been designed exclusively for use with an assoicated fiber scope or electronic scope, and a given common light source operating in a particular mode for the fibre scope, or a video processor and light source for the electronic scope, cannot be used widely with different systems.

Even in an electronic scope it is necessary to employ a different signal process in each of the different colour imaging systems, and a respective video processor and light source are required.

Therefore, as disclosed for example in the Gazette of Japanese Patent Laid Open No. 243625/1985, it has been suggested that a system be used wherein an imaging adaptor is connected to a fibre scope to display an image on a colour monitor picture surface.

In the above-mentioned prior art example, if the imaging adaptor is connected, an electronic scope employing a field sequential system colour display can be formed, and, in the event that it is connected to a control apparatus (integrating a video processor and light source), a colour display by a field sequential system can be made.

In the above-mentioned system there is a defect in that a colour mosaic type electronic scope cannot be connected for use. Also, as the system can be applied only to field sequential systems in the case of display of a moving object, it cannot be selected and used (though it is desirable), and it may be necessary to resort to fitting a colour mosaic type electronic scope.

One object of the present invention is to provide an endoscope imaging system which can be used with various colour imaging means in different colour imaging systems, by providing an endoscope which can be employed with a variety of colour imaging systems adapted to a specific use.

Embodiments of the endoscope apparatus in accordance with the present invention have the features set out in the characterising part of Claims 1 to 5.

Other features and advantages of the present invention are defined in the dependent Claims.

The invention will now be described with reference to the drawings, in which:-

Figures 1 to 8 relate to a first exemplary embodiment of the present invention:

Figure 1 being a perspective view showing the entire range of system capabilities of the first embodiment;

Figure 2 is a block schematic diagram showing the formation of a video processor for the first embodiment;

Figure 3 is a fragmentary schematic detail view showing the formation of an electronic scope using a colour mosaic filter;

Figure 4 is a fragmentary schematic detail view showing the formation of a fibre scope fitted with a field sequential type TV camera;

Figure 5 is a fragmentary schematic detail view showing the formationof a fibre scope fitted with a TV camera using a colour mosaic filter;

Figure 6 is a fragmentary schematic formation detail view showing the formation of a fibre scope;

Figure 7 is a block schematic circuit diagram showing the formation of a field sequential video processor;

Figure 8 is a block schematic circuit diagram showing the formation of a colour mosaic type video processor;

Figures 9a and 9b are exploded perspective views showing alternative light source and signal connector means;

Figure 10 is a block schematic circuit diagram showing details of an output circuit using a common signal converting stage;

Figure 11 is an explanatory view showing a connected scope sensing means;

Figure 12 is a block schematic circuit diagram of the full apparatus in a third exemplary embodiment of the present invention;

Figure 13 is a perspective view showing a connector means forming part of the third exemplary embodiment;

Figure 14 is a block schematic circuit diagram showing an output circuit provided with a signal processing function as used in a fourth exemplary embodiment of the present invention;

Figure 15 is a block schematic circuit diagram showing a modification of the fourth embodiment detail shown in Figure 14;

Figure 16 is a block schematic circuit diagram of a fifth exemplary embodiment of the present invention;

Figure 17 is a perspective view showing a connector means for use in the fifth exemplary embodiment;

Figure 18 is a partial block schematic diagram showing in magnified detail the form of a light source as used in the fifth exemplary embodiment;

Figure 19 is a block schematic diagram of an image processor as used in the sixth exemplary embodiment of the present invention;

Figure 20 is a detailed view of the light source used in the sixth embodiment;

Figure 21 is a block schematic circuit diagram of the essential features of the timing and associated sections of the image processor used in the seventh embodiment;

Figure 22 is an explanatory view showing the structure of a rotary filter forming a part of the light source used in the seventh embodiment;

Figure 23 is a perspective view showing details of the connector arrangement used in the seventh embodiment;

Figure 24 is a block schematic circuit diagram showing a field sequential type processor circuit as used in the seventh embodiment;

Figure 25 is a perspective view of a rotary filter used in the eighth embodiment of the present invention;

Figure 26 is an enlarged perspective view showing a fragmentary detail of the rotary filter means as used;

Figure 27 is a perspective view of the eighth embodiment;

Figure 28 is a perspective view of a modification of a rotary filter used in the eighth embodiment of the present invention;

Figure 29 is a sectional detail view of part of the filter shown in Figure 28;

Figure 30 is a perspective view showing the details of the ninth embodiment of the present invention;

Figure 31 is a block schematic circuit diagram of the ninth embodiment as used with a field sequential type electronic scope;

Figure 32 is a block schematic circuit diagram of the ninth embodiment as used with a mosaic type electronic scope;

Figure 33 is a block schematic circuit diagram showing a modification of the light source used in the ninth embodiment;

Figure 34 is a block schematic circuit diagram of a video processor in the tenth embodiment of the present invention;

Figure 35 is a block schematic circuit diagram of a video processor in the eleventh embodiment;

Figure 36 is an explanatory perspective view of the eleventh embodiment;

Figure 37 is a perspective view showing a modification of the eleventh embodiment;

Figure 38 is a block schematic circuit diagram of an image processor in a modification of the eleventh embodiment;

Figure 39 is a block schematic circuit diagram of an image processor in another modification of the eleventh embodiment;

Figure 40 is a perspective view showing an image processor in the twelfth embodiment of the present invention;

Figure 41 is a perspective view of the twelfth embodiment seen in a direction different from that in Figure 40;

Figure 42 is a block schematic circuit diagram of an electrical system used in the image processor of the twelfth embodiment;

Figure 43 is a fragmentary perspective view showing parts of a fitting port in the twelfth embodiment;

Figure 44 is a side view of a connecting part used in the twelfth embodiment;

Figures 45 and 46 are perspective views each showing an image processor with a separate pre-processor unit fitted and detached, respectively, in the thirteenth embodiment of the present invention;

Figure 47 is a perspective view showing an image processor with a fittable pre-processor in a modification of the thirteenth embodiment;

Figure 48 is a perspective view showing details of the fourteenth embodiment of the present invention;

Figure 49 is a block schematic circuit diagram of the fourteenth embodiment;

Figure 50 is a block schematic circuit diagram of the pre-processor circuit used in the fourteenth embodiment;

Figure 51 is a circuit detail of the post-processor circuit shown in Figure 50, for the fourteenth embodiment;

Figure 52 is a perspective view showing that a mosaic type endoscope apparatus can be used separately in the fourteenth embodiment;

Figure 53 is a perspective view showing a control apparatus of the image processor in the fifteenth embodiment of the present invention;

Figure 54 is a block schematic circuit diagram of the system used in the sixteenth embodiment of the present invention;

Figure 55 is a perspective view of the seventeenth embodiment of the present invention;

Figure 56 is a perspective view showing how the control apparatus in the seventeenth embodiment is formed as a detachable structure;

Figure 57 is a block schematic circuit diagram of the seventeenth embodiment;

Figure 58 is a perspective view showing a jointing means of a control apparatus in a modification of the seventeenth embodiment;

Figure 59 is a perspective view showing a jointing means of a control apparatus in the eighteenth embodiment of the present invention;

Figure 60 is a perspective view showing a jointing means of a control apparatus in the nineteenth embodiment of the present invention;

Figure 61 is a fragmentary sectional view showing in magnified detail a jointing part used in the nineteenth embodiment;

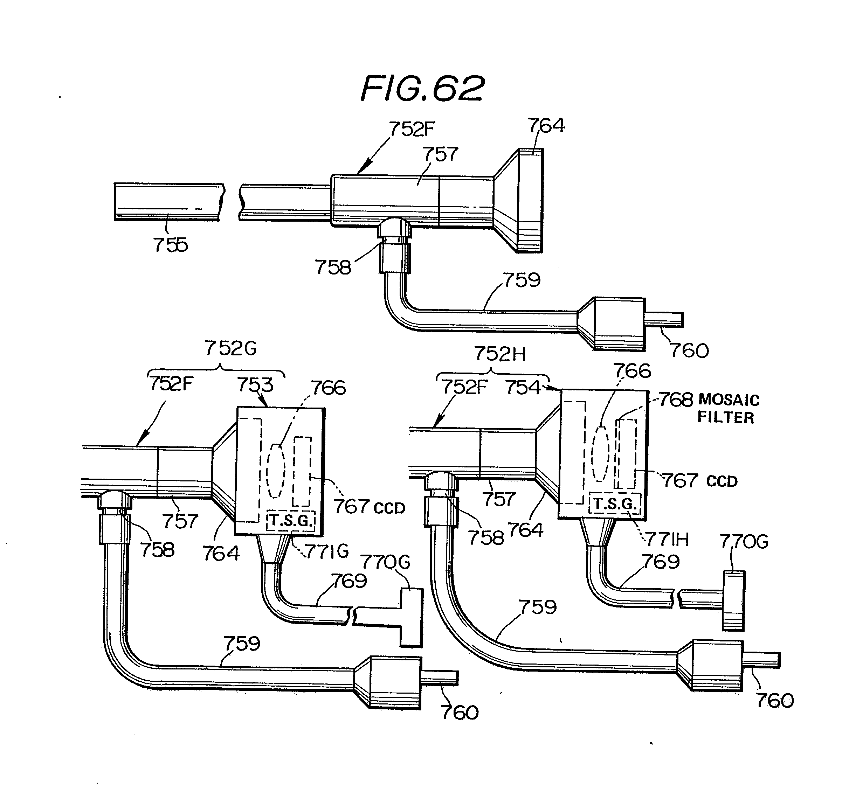

Figure 62 is a set of side views showing a rigid endoscope which can be used for the system, for example, in the first embodiment, and of rigid endoscopes fitted with externally fitted cameras; and

Figure 63 is a sectional schematic view of a rigid endoscope structure

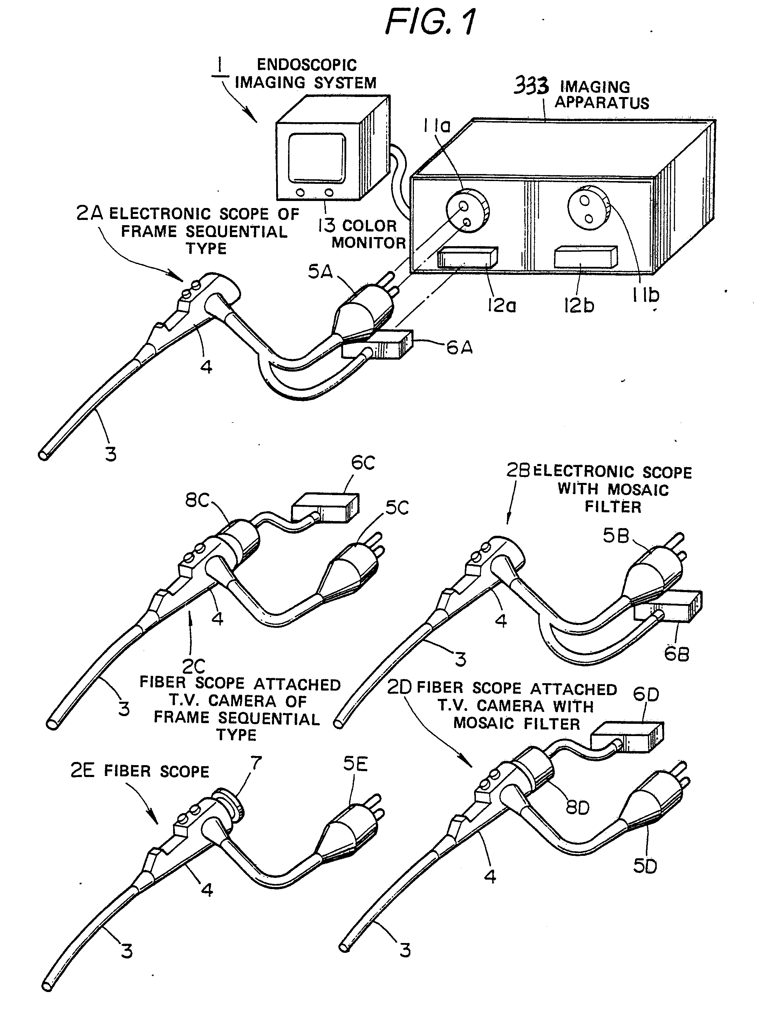

As shown in Figure 1, the endoscope image system 1 of the first embodiment has an image processor apparatus 3 which can be connected to scope of various types (endoscopes 2A,2B,2C,2D and 2E).

The scopes are of five kinds, as shown in Figure 1, that is: a frame or field sequential type electronic scope 2A; an electronic scope 2B using a colour mosaic filter (which shall hereinafter be mentioned as a colour mosaic type electronic scope or an electronic scope having a mosaic filter); a fiber scope 2C externally fitted with a field sequential type TV camera (which shall be mentioned as a fibre scope fitted with a sequential type TV camera); a fibre scope 2D fitted with an external colour masaic type TV camera (which shall be mentioned as a fibre scope fitted with a colour mosaic type TV camera); and a fibre scope 2E. Each of the scopes, 2A to 2E has an elongate insertable part 3, an operating part 4 at the rear end of the insertable part 3, a universal cord extended out of this operating part, and a respective light source connector 5A to 5E provided at the cord end. In this case, in the frame or field sequential type electronic scope 2A, and colour mosaic type electronic scope 2B, in addition to the light source connectors, 5A and 5B respectively, respective signal connectors 6A and 6B are provided on the ends of the above-mentioned universal cords. In the fibre scope 2C fitted with a frame sequential type TV camera and fibre scope 2D fitted with a colour mosaic type TV camera, a field sequential type TV camera 8C and colour mosaic type TV camera 8D are respectively fitted to the eyepiece part 7 that is visible on the fibre scope 2E. Two sets of connector sockets are provided, for example, on the front surface of a housing of an image or video processor 333, so that the respective scopes may be set in a usable state by connecting the requisite connector, 5A and 6A, 5B and 6B, 5C and 6C, 5D and 6D or 5E alone, of the respective scopes 2A to 2E. These connector sockets consist of a sequential type light source connector socket 11a, a sequential type signal connector socket 12a, a white light source connector socket 11b, and a colour mosaic type signal connector socket 12b. The light source connectors 5A and 5C are of the same form, since they respectively serve for the sequential type electronic scope 2A and the fibre scope 2C fitted with a sequential type TV camera (these two scopes 2A and 2C shall also be refered to as sequential type scopes) are of such form as can be connected respectively to the frame or field (as the case may be) sequential type light source connector socket 11a. The respective signal connectors 6A and 6C are of the same form, for the sequential type electronic scope 2A and the fibre scope 2C, fitted with sequential type TV cameras, that is, the sequential type scopes 2A and 2C are of such form as can be connected to the sequential type signal connector 12a adjacent the lower side of the sequential type light source connector socker 12a.

The light source connector 5B of the colour mosaic type electronic scope 2B, the light source connector 5D of the fibre scope 2D fitted with the colour mosaid type TV camera (these two scopes 2B and 2D shall also be referred to also as mosaic type scopes) and the light source connector 5E of the fibre scope 2E may be respectively connected to the white light source connector socket 11b, so these connectors 5B,5D and 5E are made in the same form. Also, so that the signal connector 6B of the colour mosaic type electronic scope 2B and the signal connector 6D of the fibre scope 2D fitted with a colour mosaic type TV camera may be connected to the colour mosaic type signal connector 12b adjacent the lower side of this white light source connector socket 11b, these connectors 6B and 6D are made in the same form.

When the fibre scope 2E is connected and used, the observation is made by a naked eye. When another type of scope, 2A,2B,2C or 2D is used, the reproduced image can be displayed on a television monitor, preferably a colour monitor 13, connected to the signal output end of the video processor 333 provided as an image processor.

The light source connectors 5A to 5E in the respective scopes are provided with light guide connectors, air and/or water feed connectors as required, which can be connected via the connector sockets 11a and/or 11b.

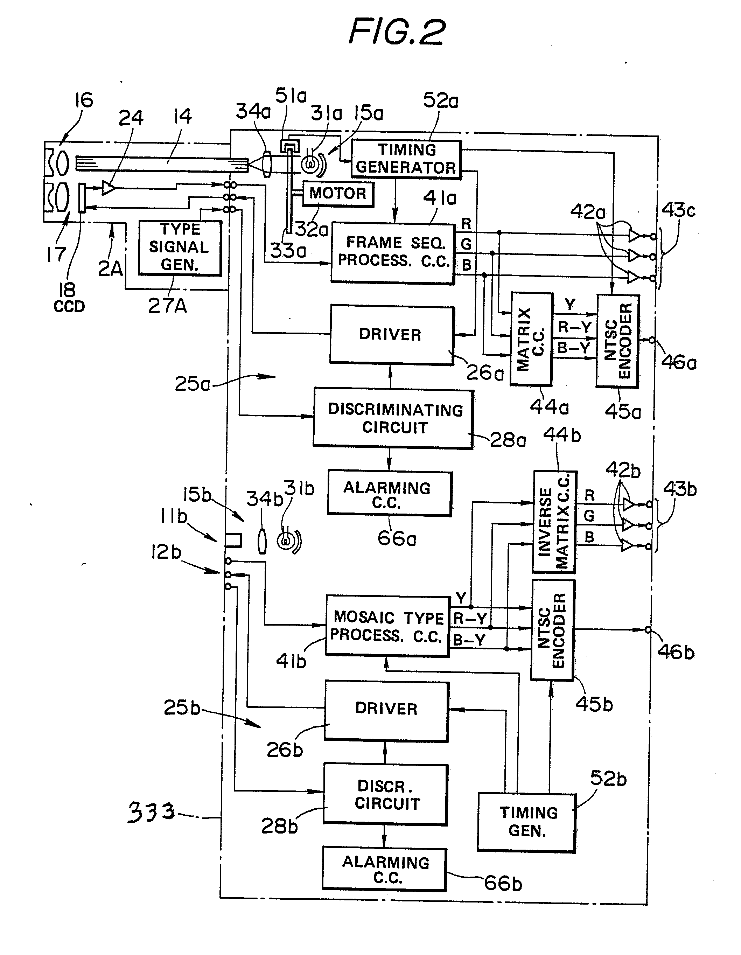

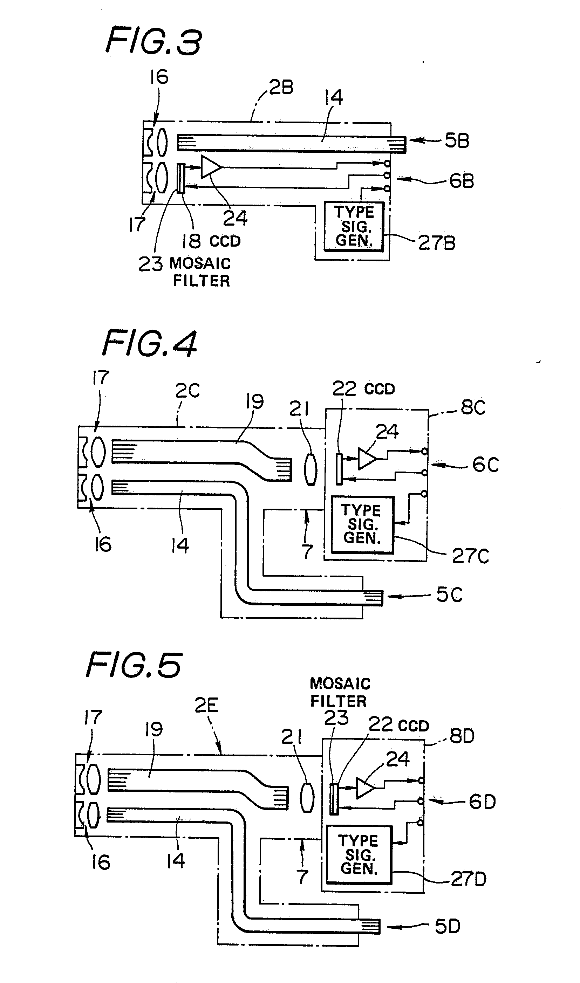

The internal formations of the respective scopes 2A to 2E are shown respectively in Figures 2 to 6. A light guide 14 for transmitting anilluminating light is inserted through each scope 2 so that illuminating light from the light source 15a or 15b within the video processor imaging apparatus 333 can pass via the entrance end-face to be transmitted to the exit end-face at the distal tip of the insertable part and pass out to illuminate the object through a light distributing lens arranged in front of this exit end-face.

An image forming objective 17 is arranged in the tip of the insertable part 3 of each scope, 2A to 2E. In the field sequential type or colour mosaic type electronic scope 2A or 2B, a CCD element 18 is arranged in the focal plane of this objective 17. On the other hand, in the fibre scope 2E, or the fibre scope 2C or 2D fitted with a TV camera 8C or 8D respectively, the entrance end-face of the image guide 19 is arranged to be present in the focal plane of the objective 17.

An eyepiece 21 is arranged opposed to the exit end-face of the above-mentioned image guide 19. In the fibre scope 2E, an observation can be made with a naked eye brought close to the eyepiece part 7.

If the fibre scope 2E fitted in the eyepiece part 7 is to be used, the sequential type TV camera 8C or colour mosaic type TV camera 8D may be used, a CCD element 22 is arranged in the image plane of the eyepiece lens 21 through an image forming lens (not illustrated). A colour mosaic filter 23 is arranged on the front face the imaging surface of the CCD element 18 or 22 used in the colour mosaic type electronic scope 2B or colour mosaic type TV camera 8D. The optical image formed on the imaging surface is photo-electrically converted by the CCD element 18 or 22, and the resultant signal is amplifed by a pre-amplifer 24, to be transmitted to the signal connector 6A,6B,6C or 6C via a signal transmitting line and is fed into a video processor stage 25a or 25b through the signal connector socket 12a or 12b to which said connector is connected. Also, a CCD driving clock applies a pluse train from a driver 26a or 16b, forming part of the video processor apparatus, to control the CCD element 18 or 22. Here, the video processor stage 25a represents the entire frame sequential type signal processing system. The video processor stage 25b represents the entire mosaic type signal processing system.

Type signal generating circuits 27A,27B,27C or 27D emit respective scope discriminating type signals via the signal connector in all scopes other than the fibre scope 2E, and the particular type signal is identified by a discriminating circuit, 28a or 28b.

The video processor apparatus 333 to which any of the above-mentioned scopes can be connected contains two separate light sources, 15a and 15b, and two separate video processor stages, 25a and 25b, as shown in Figure 2.

One light source 15a is of a frame or field sequential type. A white light from a source lamp 31a is made to produce a sequence of differently coloured illuminating light R,G and B by a rotary filter 33a that is rotated by a motor 32a, condensed by a condenser lens 34a, and is then fed to the entrance end-face of the light guide 14 fitted to the connector socket 11a.

The other light source 15b is a white light source. Light from a white lamp 31b is condensed by a condenser lens 34b, led to a white light source connector socket 11b and fed to the entrance end-face of a light guide 14 fitted to this connector socket 11b.

One video processor stage 25a is for field or frame sequential type signal processing. The signal input to the input terminal of the sequential type signal connector socket 12a is fed into a sequential type processor circuit stage 41a, and the signals respectively formed into images under the illumination by lights of the respective wavelengths R,G and B are fed out as individual colour signals R,G and B. The above-mentioned signals R,G and B thus feed out three primary colour signals R,G and B.

These colour signals, R,G and B, are transmitted through a matrix circuit 44a to produce a luminance signal Y and two colour-difference signals, R-Y and B-Y, which are then fed into an NTSC encoder 45a to be converted to a composite video signal of the NTSC system type, which is emitted at an NTSC output terminal 46a.

A rotary position sensor 51a is provided for detecting the rotary position, positioned at a point on the outer periphery of the rotary filter 33a forming the field sequential system light source 15a. By its output, the timing of the clock of the timing generator 52a is synchronised wiht the rotation of the rotary filter 33a, and the timing of the frame sequential type processor circuit 41a is controlled by the output of this timing generator 52a.

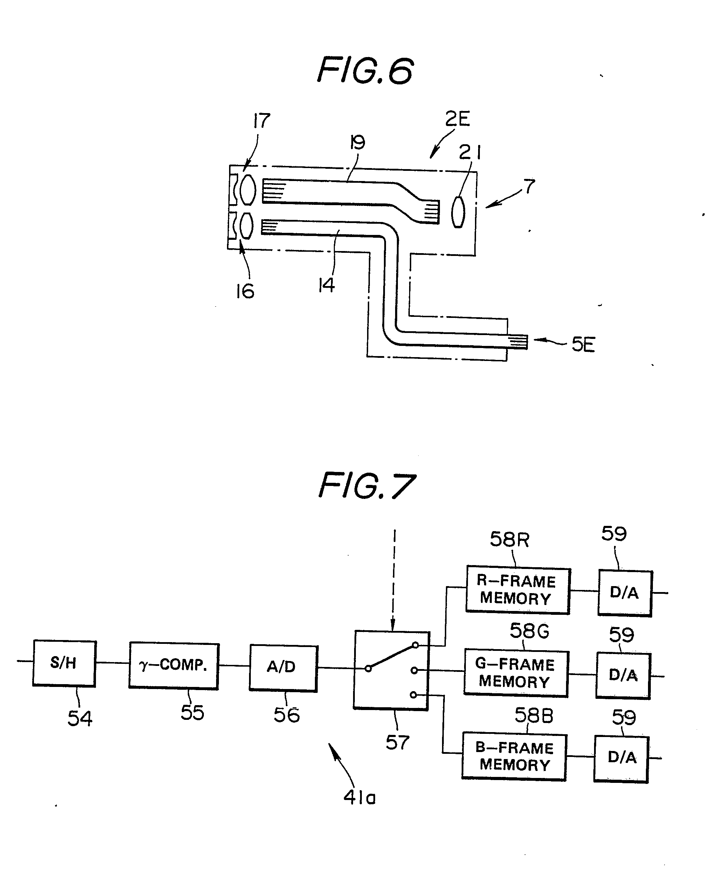

This frame sequential type processor circuit 41a is formed as shown in Figure 7, for example.

The signal input from a pre-amplifier is fed into a sample holding circuit 54 to be held until it is γ-corrected in a γ-correcting circuit 55 and then converted to a digital amount in an A/D-converter 56. Then, the signals imaged under the frame sequential illumination of R,G and B are passed selectively through a multiplexer 57 switched by the signal of the timing generator 52a and are written into an R-frame memory 58R, a G-frame memory 58G and a B-frame memory 58B. The signal data written into these respective frame memories,

5

58R,58G and 58B, is read out simultaneously, converted to respective analogue colour signals, R,G and B by individual D/A-converters 59, and is then fed out to the matrix circuit 44a (Figure 2).

The signal imaged by the CCD elements 18 or 22, through the colour mosaic type signal connector 12b, is fed into a colour mosaic type processor stage 41b to provide a luminance signal Y and two colour-difference signals, R-Y and B-Y. These signals are input into an NTSC encoder 45b and converted to a composite video signal which is fed from the NTSC output 46b. Also, these signals form an input into an inverse matrix circuit 44b and are converted to colour signals R,G and B, and the three primary colour signals R,G and B are fed out from three respective primary colour outputs 43b, each fed via a separate buffer 42b forming drivers.

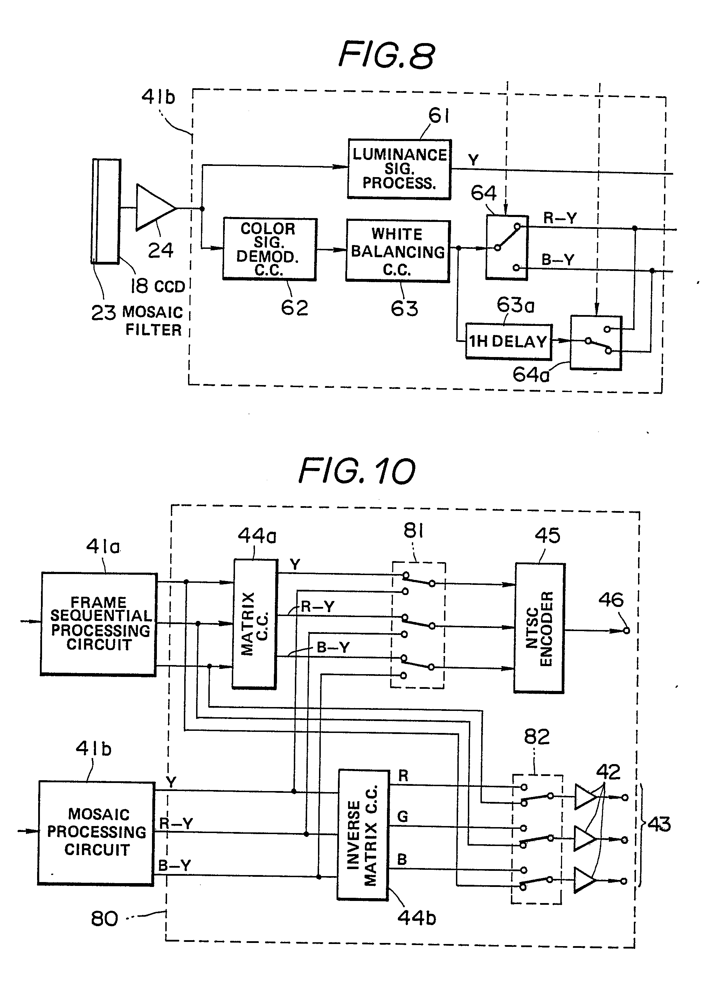

In the colour mosaic type processor stage 41b, for example as shown in Figure 8, the signal from the CCD element 18 (or 22) is amplifed by a pre-amplifier 24 and produces a luminance signal Y through a luminance signal processing circuit 61. The above-mentioned signal is also input into a colour signal demodulating circuit 62. Colour-difference signals R-Y and B-Y are produced in each horizontal line in a time sequence and are compensated for white balance in a white balancing circuit 63. One of the signals is input directly into an analogue switch 64 and the other is delayed by one horizontal line by a 1H delay line 63a and input into an analogue switch 64a to obtain colour signals R-Y and B-Y by switching control signals of a timing generator 52b (Figure 2).

The respective timing generators 52a and 52b apply signals respectively to the drivers 26a and 26b, and to the encoders 45a and 45b, and control to process the signal synchronised with the driving pulse used to read out signals from the CCD element 18 or 22. In this case, the timing generator 52a is synchronised with the rotary filter 33 by the output of the rotary position sensor 51a. The NTSC encoders 45a and 45b are formed with individual buffers included.

Each of the type signal generating circuit, 27A,27B,27C and 27D is formed, for example, by connecting a resistance or the like of a different resistance value between two terminals. On the other hand, the connected scope of any resistance value between the two terminals can be discriminated by using a comparator or the like in the discriminating circuits 28a and 28b.

For example, in case the signal connector 6B or 6D of the colour mosaic type electronic scope 2B or the fibre scope 8D fitted with the colour mosaic type TV camera is connected to the frame sequential type signal connector socket 12a, it will be discerned that it is not of a resistance value appropriate for the frame sequential type, a warning circuit 66a will be operated by the discriminated signal, and it will be made known to the user by a warning buzzer or a flickering luminescence diode ED.

Also, in case the connector 6A of the frame sequential type electronic scope 2A or the connector 6C of the fibre scope 2C fitted with the frame sequential type TV camera is connected to the colour mosaic type signal connector receptacle 12b, this will be detected by the discriminating circuit 28b and a warning will be given by a warning circuit 66b.

On the other hand, when the connector 6A or 6C of the frame sequential type scope 2A or 2C is connected to the frame sequential type signal connector socket 12a, it will not be warned. (If the connection is right, it may be displayed by lighting an LED). Likewise, if the connector 6B or 6D of the colour mosaic type scope 2B or 2D is connected to the colour mosaic type connector socket 12b, the warning circuit 66b will not operate. (This fact may be displayed by lighting an LED whose position or colour is different from that used for discriminating and warning that the connection is right). Also, in case two signal connectors are simultaneously connected to both signal connector sockets 12a and 12b, warning may be given. Also, a light source connector connection sensing means may be provided within the frame sequential type light source connector socket 11a so that, in case the connector 5E of the fibre scope 2E is connected, it may be made known to be a mis-connection. That is to say, it is possible to warn in case the connector 5E is connected to the connector socket 11a and no connector is connected to the signal connector socket 11a and 11b.

In the thus formed first embodiment, the sequential type scope light source 15a and sequential type video processor stage 25a are provided, together with colour mosaic type scope light source 15b, colour mosaic type video processor stage 25b, and connecting means for the respective scopes, and, even if any of the sequential type scopes 2B and 2C and colour mosaic type scopes 2B and 2D is connected, the illumination light feed and signal process corresponding to the connected scope can be produced and so produce an object image via the connected scope to be colour-displayed.

In cases where the fibre scope 2E is used, its light source connector 5E can be connected to the white light source connector socket 11b, and thus an observation can be made by the naked eye.

If a wrong type of scope is connected to two sets of connector sockets, 12a and 12b, the wrong connection will be sensed by the discriminating circuit 28a or 28b, and a warning given by the warning circuit 66a or 66b.

Therefore, according to this first embodiment, when one imaging apparatus 3 is provided, a scope having a different colour imaging system can be used, and even the basic fibre scope 2E can be simultaneously used. In the event of a wrong connection being made, a warning will be given. Therefore the apparatus is convenient to use.

If the connector 6 and connector socket 12 are made in forms that differ between the sequential type and mosaic type, it is apparent that the mis-connection cannot pass unnoticed, but will be eliminated.

The signals processed for the above-mentioned two-colour imaging systems have the same type of output, i.e. they are made to coincide with the three primary colour outputs or NTSC system video signals, and therefore a common colour monitor 13 can be used. (This colour monitor may respond either to the three basic primary colour signals, or to an NTSC system standard video signal).

6

If the TV camera 8C or 8D is fitted to the fibre scope 2E, the visual picture image will be displayed on the colour monitor 13. If the TV camera 8C or 8D is removed, the fact can be indicated by displaying an indication of the removed state on the picture screen of the colour monitor 13.

According to the system of this first embodiment, a colour imaging system selected as that best adapted to the required use can be readily used.

For example, if a high resolution is required, then, by using a frame sequential type colour imaging scope 2A or 2C, a high resolution colour picture can be displayed on the colour monitor 13.

On the other hand, if the use is to be near a moving object, the mosaic filter type colour imaging scope 2B or 2D is used and a steady colour image having little colour displacement can be obtained which is free of the streaking effects that are associated with sequential colour systems.

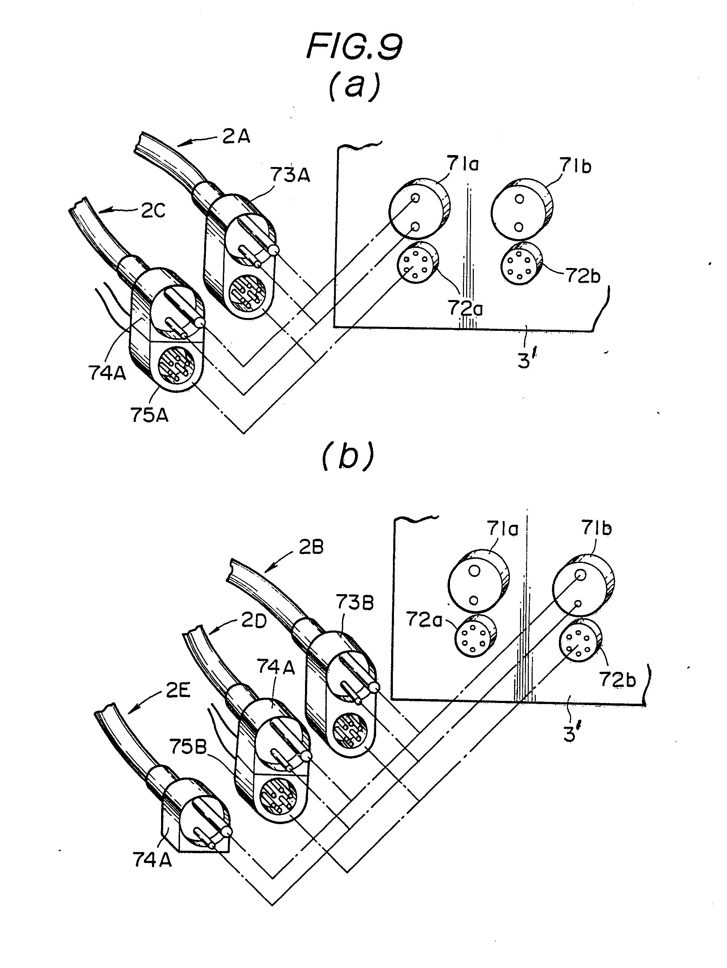

Figure 9 shows how modification of a connector and connector socket arrangement may be achieved.

An imaging apparatus 3' is proved with a round frame sequential type light source connector socket 71a adjacent its signal connector socket 72a, and a white light source connector socket 71b adjacent its colour mosaic type signal connector socket 72b, separated on a housing front surface or the like. Both connector sockets 71a and 71b have the same form, and the sockets 72a and 72b are also of the same form, but this differs from that of socket 71a and 71b.

As shown in Figure 9a, a frame sequential type scope 2A is provided with a connector 73A integrating a light source connector part and signal connector part so as to be simultaneously connectible to the frame sequential type light source connector socket 71a and signal connector socket 72a.

In the same manner, as shown in Figure 9b, a colour mosaic type scope 2B is provided with a connector 73B connectible to the above-mentioned white light source connector socket 71b and colour mosaic type signal connector socket 72b.

As shown in Figure 9b, a fibre scope 2C fitted with a frame sequential type TV camera can be made with a connector in the same form as that of the connector 73A of the frame sequential type electronic scope 2A, when a light source connector 74A and signal connector 75A are combined with each other and can be used as connected to the sequential type connector sockets 71a and 72a.

Also as shown in Figure 9b, a fibre scope 2D fitted with a colour mosaic type TV camera can be made in the same form as the connector 73B of the colour mosaic type electronic scope 2B, when a light source connector 74B and signal connector 75B are combined in a form connectible to the white light source connector socket 71b and signal connector socket 72b.

When connected to the white light source connector socket 71b, the light source connector 74A of the fibre scope 2E can feed a white light toward the light guide of the fibre scope 2E and observation can be made with the naked eye.

In case a connection different frm the connections shown in Figures 9a and b is made, as explained in the first embodiment by the connection of the signal connector, the signal of the type signal generating circuit is detected by a discriminating circuit and a warning is issued.

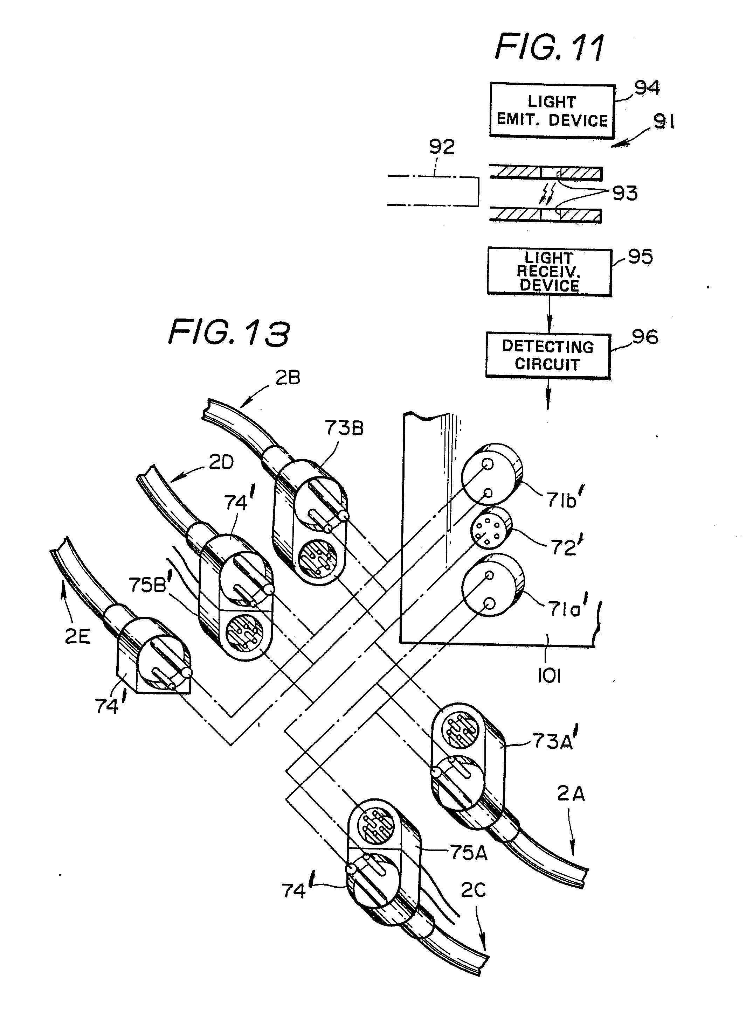

Figure 10 shows an essential part of a video processor stage in the second embodiment of the present invention.

In this second embodiment, an output circuit 80 (provided with a signal converting function) has in common the output arrangement of the video processor stages 25a and 25b shown in the first embodiment.

That is to say, in Figure 2, a 3-pole/2-way input selector switch 81 is provided between the output ends of a matrix circuit 44a and an NTSC encoder 45, and a 3-pole/2-way output switch 82 is provided also between the output ends of an inverse matrix circuit 44b and respective buffers 42 each forming a driver.

In the input selector switch 81, when contact is to one side, the signals of the matrix circuit 44a will be led to the common NTSC encoder 45 and will be made into an NTSC system video signal in this NTSC endoder 45, so that the video signal will be fed out from a common NTSC output 46. When the other switch contact position is selected, the signals of a mosaic type processor circuit 41b will be led to the NTSC encoder 45 and will be output from a common NTSC output 46.

The other selector switch 82 is so arranged that, when the frame sequential side is selected, the output signals of the frame sequential processor circuit 41a will pass through the three buffers 42 forming drivers, and three primary colour signals will be available from common R,G and B outputs 43. When the mosaic type processor circuit side is selected, three primary colour signals R,G and B transmitted through the inverse matrix circuit 44b will be available from the common R,G and B output 43.

The selector switches 81 and 82 can be respectively switched manually or as operatively connected. Also, the type signal output from the scope to which the selector switches 81 and 82 are connected is monitored as shown in Figure 2 by a respective discriminting circuit 28a or 28b. By this discriminated signal, the selector switches 81 and 82 can be switched to a processor circuit 41a or 41b to process the signal in the manner corresponding to that required for the connected scope.

If the selector switches 81 and 82 are formed by analogue switches or the like, they will be able to be automatically switched by a connection sensing apparatus 91, as shown in Figure 11.

For example, the frame sequential type connector is provided with a discrimating pin 92, which is not provided in the mosaic type. The frame sequential type connector socket is provided with a recess in which this pin 92 can be engaged. Opposed horizontal holes 93 are provided on both sides of this recess, and a light emitting device 94 such as an LED and a light receiving device 95 such as a photo-diode are oppositely arranged with the output of the light receiving device 95 feeding into a detecting or sensing circuit 96. When the pin 92 is engaged in the recess, the light from the light emitting device 94 will be intercepted and the output of the light receiving device 95 will vary from "L" to "H" or the like. This output variation will be sensed by the sensing circuit 96, and the switching switches 81 and 82 will be switched so that the frame sequential side may be conductive. If the output of the light receiving device 94 is "L", the colour mosaic type process circuit side will be selected.

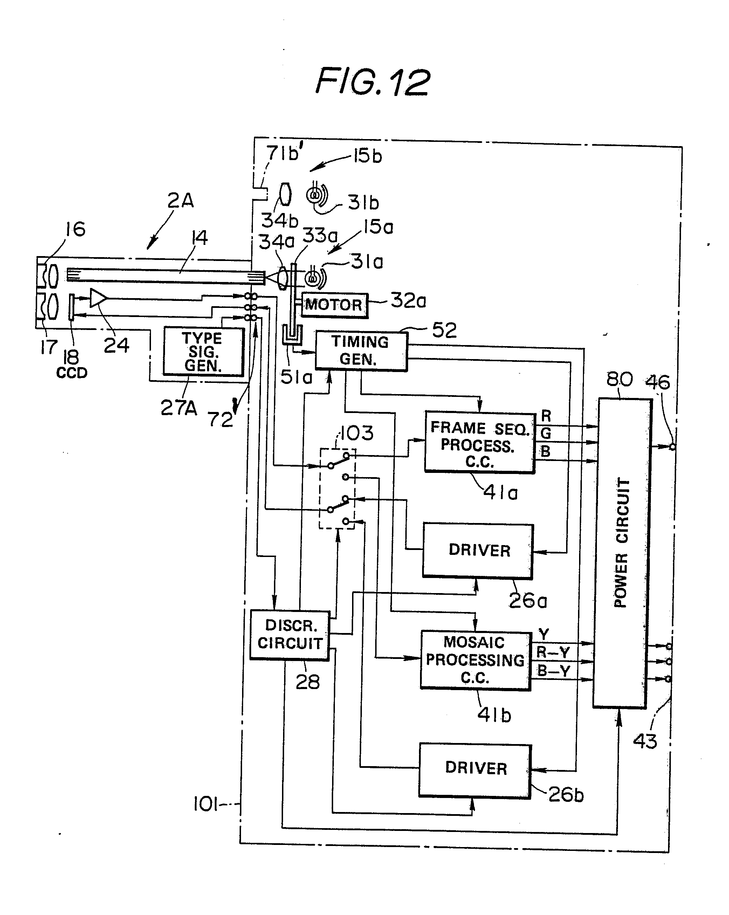

Figure 12 shows the third embodiment of the present invention.

In the imaging apparatus shown in Figure 2, the signal side input end of the electronic scope 2 is made common, whereas, in this embodiment, the output side is made common.

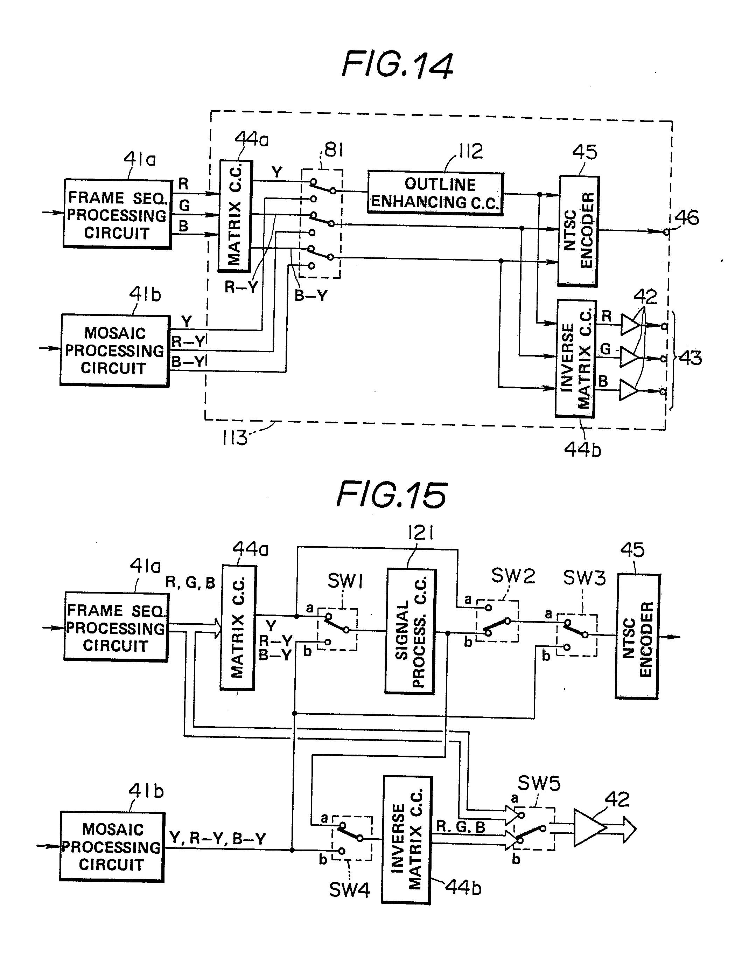

A signal connector socket 72' common with light source side connector sockets 71a' and 71b' of the video processor apparatus of this embodiment is of the form shown, for example, in Figure 13. Together with a connector 73A' of the sequential type scope 2A or a connector 73B' of the mosaic type electronic scope 2B, the respective signal connector parts can be connected to the common signal connector socket 72' and the light source connector parts connected to the light source connector sockets 71a' or 71b' provided respectively below and above. The light source connector 74' and signal connector 75A' of the scope 2C fittd with the frame sequential type TV camera or the connectors 74' and 75B' of the scope 2D fitted with the mosaic type TV camera are also formed in the same manner. Further, the connector 74' of the fibre scope 2E can be connected to the white light source connector socket 71b' when required.

The internal formation of the video processor stage in the apparatus 101 is as shown in Figure 12.

The output signal of the type signal generating circuit (for example 27A) is fed into the common discriminating circuit 28 through the common signal connector socket 72' to identify the type of the connected scope in this discriminating circuit 28. As in the first embodiment, this discriminating circuit 28 controls not only both drivers 26a and 26b, but also the switching of a newly provided 2-pole/2-way selector switch 103. For example, as shown in Figure 12, if the frame sequential type scope 2A or 2C is connected, the circuit will be switched to the frame sequential side, the driving pulse of the driver 26a will be applied to the CCD element 18 through the connector, and the signal read out of the CCD element 18 will be fed into the frame sequential type processor circuit 41a.

On the other hand, if the frame sequential type scopes 2A and 2C are not connected, the mosaic type processor circuit side will be selected, and,in the case of the mosaic type scope 2B or 2D, the selector switch 103 may be switched to the mosaic type side.

The discriminating circuit 28 also feeds a control signal to a timing generator 52 made common so as to be able to cope with either system.

Also in this embodimnt, the signal through the processor circuit 41a or 41b uses an output circuit 80 shown, for example, in Figure 10. The selector switches 81 and 82 (see Figure 10) are operatively connected by the output of the discriminating circuit 28. For example, in the event of usage of the frame sequential type scope 2A or 2C being detected, the switches will be switched to the frame sequential side shown in Figure 10.

Instead of using the output circuit 80 shown in Figure 10, separate outputs may be provided for the frame sequential type and the mosaic type.

Also, when using the output circuit 80 shown in Figure 10, the selector switches 81 and 82 may be manually switched.

The other elements are of the same formation as in the above-described first embodiment. In the embodiment shown in Figure 12, instead of using separate light source lamps 31a and 31b, one only may be used (for example the lamp 31b only), and the position of this lamp 31b made movable to be switched to the position of the other lamp 31a. Then both systems will be able to be illuminated using one lamp. Also, two light source lamps 31a and 31b may be provided, diametrically opposed about the centre of a rotary plate, so that the positions of each may be exchanged (that is, the lamp 31a placed in the position of 31b) by the rotary operation and, even if one lamp is broken, the other lamp may then be used as an auxiliary lamp.

In this embodiment, as in the first embodiment, if the light source connector of the fibre scope 2E is connected to the apparatus 101, observation by the naked eye can be made.

If onle the connector 74' of the fibre scope 2E is connected to the white light source connector socket 71b', by providing means for sensing the connection, the fact that the fibre scope 2E is connected may be displayed by a monitor.

In the third embodiment, the signal connector receptacle 72' is common, but may be separate as shown in Figures 1 or 9.

Figure 14 shows an essential part of a video processor storage in the fourth embodiment of the present invention.

In this video processor stage, in the output circuit 80 having the signal converting function shown in Figure 10, there is made an output stage 113 wherein an outline enhancing signal process is made through an outline enhancing circuit 112 on a luminance signal switched by a 3-pole selector switch 81. The other selector switch 82, shown in Figure 10, is not necessary but may be provided to increase the adaptability of operational choices.

The selector switch 81 may be switched by the output of the discriminating circuit 28 shown in Figure 12 or may be manually switched.

The other elements are the same as shown in Figure 10.

According to this embodiment, the outline is enhanced in common for different luminance signals of two systems. Therefore, as compared with the case that two sets are provided for the respective systems, the number of parts can be reduced and the formation simplified so that the cost becomes lower.

In Figure 14, not only the outline enhancement (horizontal or vertical or both), but also the NTSC encoder 45, inverse matrix circuit, and coaxial cable driver 42, are used in common. Instead of the outline enhancing circuit 112, a line interpolting circuit may be provided, and an auto-gain-control circuit may be provided.

The other commonly used circuits may be such as, for example, a frame memory, stationary picture memory, colour burst generation, power source, character generator, superimposing circuit, keyboard controller and tone adjustment device.

Figure 15 shows a modification of Figure 14. That is to say, in the circuit shown in Figure 14, the line enhancing signal process is made for the signal (luminance signal) of either of the frame sequential type or mosaic type. In the signal processing part shown in Figure 15, such a signal process as the outline enhancement can be selected. Even if no signal is processed, the signal deterioration will be prevented. Therefore, switches SW1 and SW2 are provided to front and rear of the signal processing circuit 121 in the rear step of the matrix circuit 44a. The output of the mosaic type processor circuit 41b can be fed into the NTSC encoder 45 through a switch SW3 on the output side of the switch SW2. In the event of the signal being passed through the signal processing circuit 121, it is fed out from the RGB output end, to pass through a switch SW4, inverse matrix circuit 44b, and switch SW. So that the R, G and B signals of the frame sequential type processor circuit 41a may not be deteriorated by returning again to the R, G and B signals through the matrix circuit 44a and inverse matrix circuit 44b, three primary colour signals R, G and B can be output directly from the RGB output end through a switch SW5.

Whether the signal is processed (on) or not (off), the state of the respective switches SW1 to SW4 in the modification shown in Figure 15 is as shown in the logical table below:-

## LOGICAL TABLE

| Output | Signal process | SW1 | SW2 | SW3 | SW4 | SW5 |
|---|---|---|---|---|---|---|
| Frame sequential type | On | a | b | a | a | b |
| | Off | Δ | a | Δ | a | a |
| Mosaic type | On | b | b | a | a | b |
| | Off | Δ | Δ | b | b | b |

Δ represents that either side is good.

In the embodiment shown in Figure 15, the luminance signal Y and colour-difference signals R-Y and B-Y are processed, but, if desired, only the luminance signal may be processed.

In the circuit shown in Figure 10, the luminance signal and respective R,G and B colour signals may be processed in the latter steps of the respective selector switches.

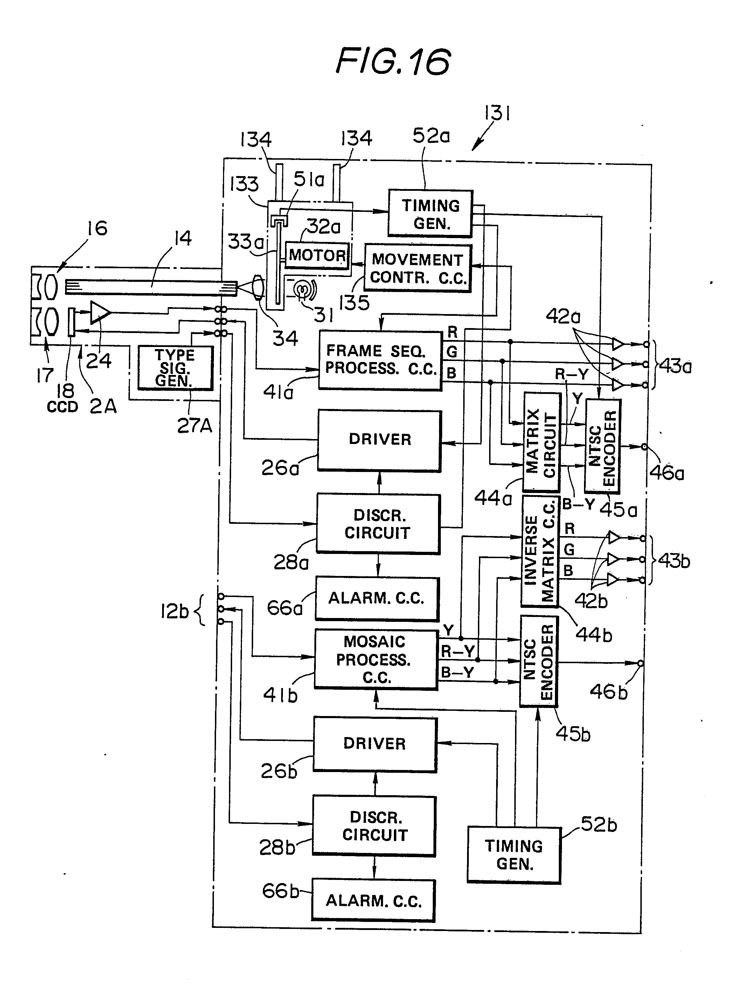

Figure 16 shows another video processor apparatus having in common the light sources and connector part of the fifth embodiment of the present invention.

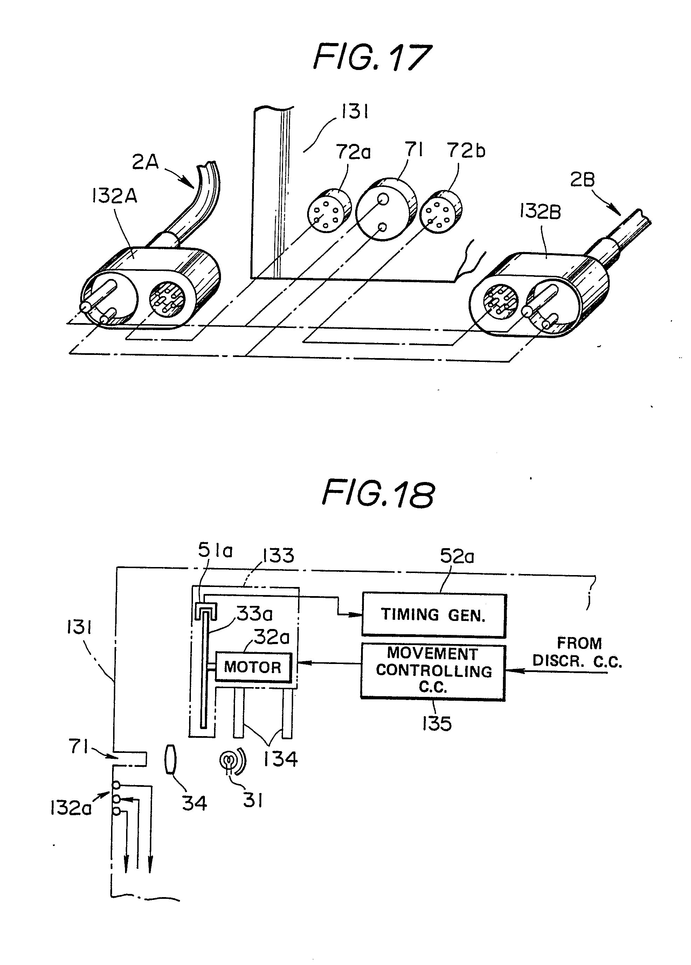

For example, on the front surface of a housing of a video processing apparatus 131 in the fifth embodiment as shown in Figure 17, a common light source connector socket 71 is provided, together with a frame sequential type signal connector socket 72a and a colour mosaic type signal connector socket 72b symmetrically arranged on both sides of the light socket.

On the other hand, the connector 132 of the field sequential type electronic scope 2A and the connector 132B of the colour mosaic type electronic scope 2B are so made that both of their light source connector parts can be fitted to the light source connector socket 71, and that the respective signal connectors can only be connected as appropriate, respectively to the field sequential type connector socket 72a and colour mosaic type connector socket 72b. Though not shown in Figure 17, the same applies for the electronic scopes 2C and 2D fitted respectively with the field sequential type and mosaic type TV cameras. The fibre scope 2E can have its connector connected to the light source connector socket 71 to make a naked eye observation.

In the video processing apparatus 131 shown in Figure 16, as magnified and shown in Figure 18, a rotary filter 133 is made movable along rails 134.

The rotary filter part 133 is set normally at one end of the rails 134. For example, as shown in Figure 18, when the rotary filter 33a is withdrawn, from the light path between the light source lamp 31 and lens 34, a constant white light source will be formed. If the rotary filter 133 is moved from this state to the lower ends of the rails 134 (as shown in Figure 16) it will be interposed in the light path and a frame sequential light source will be formed.

Now, the rotary filter part 133 is controlled in movement by the movement controlling circuit 135, which is operated by a signal from the discriminating circuit 28a. In this embodiment, when the frame sequential type scope is identified by the type signal as being from a type signal generating circuit 27A or 27C, then the discriminating circuit 28a emits a movement controlling instruction to a movement controlling circuit 135, and a rotary filter 133 will be moved from the withdrawn state shown in Figure 18 to the operative state shown in Figure 16.

If the connector of a mosaic type scope 2B or 2D is connected, then the rotary filter 133 will not be moved, and a white light will be fed out. Also, in the event of a fibre scope 2E being fitted, this will also apply and a white light will be fed to the connector of the fibre scope.

If the frame sequential type scope 2A or 2C is fitted and is then removed, the rotary filter part 133 will be withdrawn back up out of the light path.

The other elements are the same as those of the apparatus shown in Figure 2.

According to this fifth embodiment, as a common light source is used without having to provide two light source assemblies, both the field sequential type and mosaic type scope can be selected for use as required. Also, in case the connector of the fibre scope is to be connected, it is ensured that a mis-connection (such as connecting to the field sequential type side by mistake if there were two light source connector sockets) is totally avoided.

The rotary filter 133 may be manually moved at any time, if required.

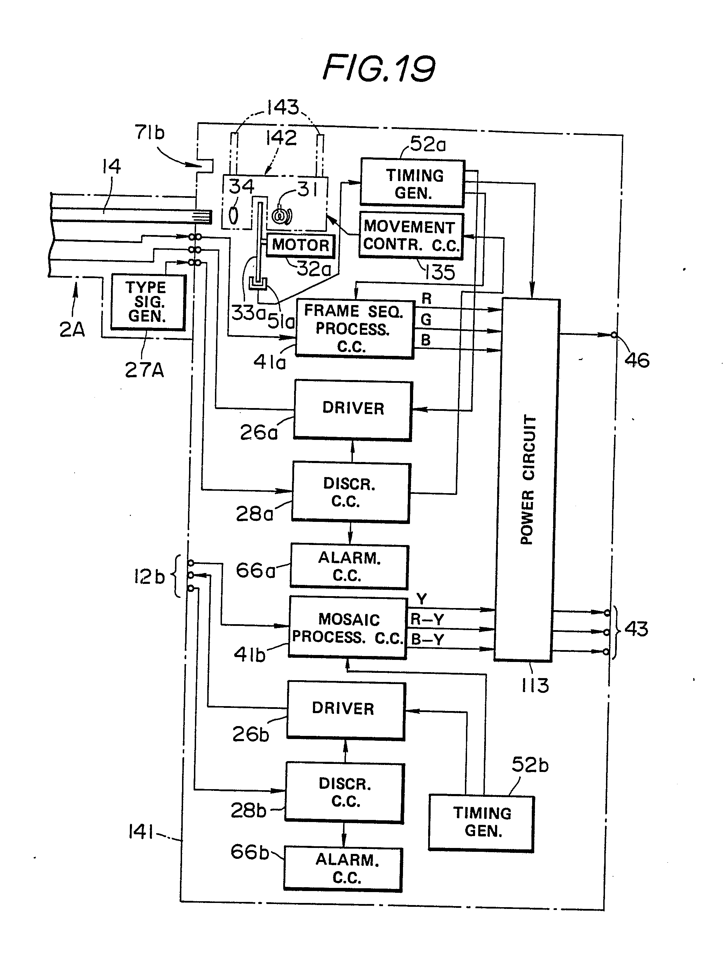

Figure 19 shows a video processor unit 141 in the sixth embodiment.

In the fifth embodiment, the rotary filter 13 is made movable. In this sixth embodiment, a common light source 142 is made movable along rails 143.

The connector socket, for example on the front face of the unit 141 of this embodiment, has the format shown in Figure 9 and the scope side connector is also of the form shown in that Figure.

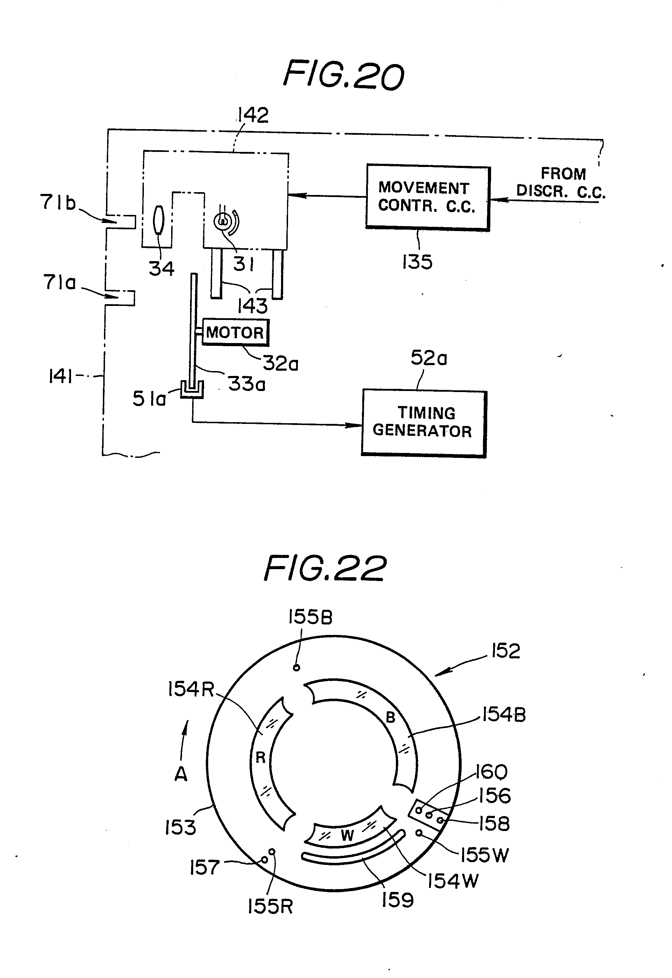

As shown in Figure 20, the light source 142 is movable within the unit 141, and is normally aligned with the white light source connector socket (represented by the same reference numeral 71b as shown in Figure 9). When a field sequential type scope 2A or 2C is connected, then, as in the fifth embodment, it will be detected by the type signal in the discriminating circuit 28a and, in this case, the light source 142 will be moved (downward in Figure 20 or horizontally in Figure 9) by the movement controlling circuit 135 to be aligned with the frame sequential type connector socket 71a (as shown in Figure 19), and the sequential illuminating lights of R,G and B passed through the rotary filter 33 will be fed to the sequential type light source connector.

As shown in Figure 19, the unit 141 of this embodiment differs from the one shown in Figure 16 and uses a common output circuit 113 - details of which are shown in Figure 14.

The other elements are the same as those shown in Figure 16, and have substantially the same operation and effect.

In the sixth embodiment, the connector socket can be moved together with the light source 142. In such a case, it will not be moved when a mosaic type scope 2B or 2D is connected, but will be moved when a sequential type scope 2A or 2C is connected. It will not be moved in the case of a fibre scope 2E being connected. In this embodiment, there is only the one connector socket, and a manually movable structure can be employed if so desired.

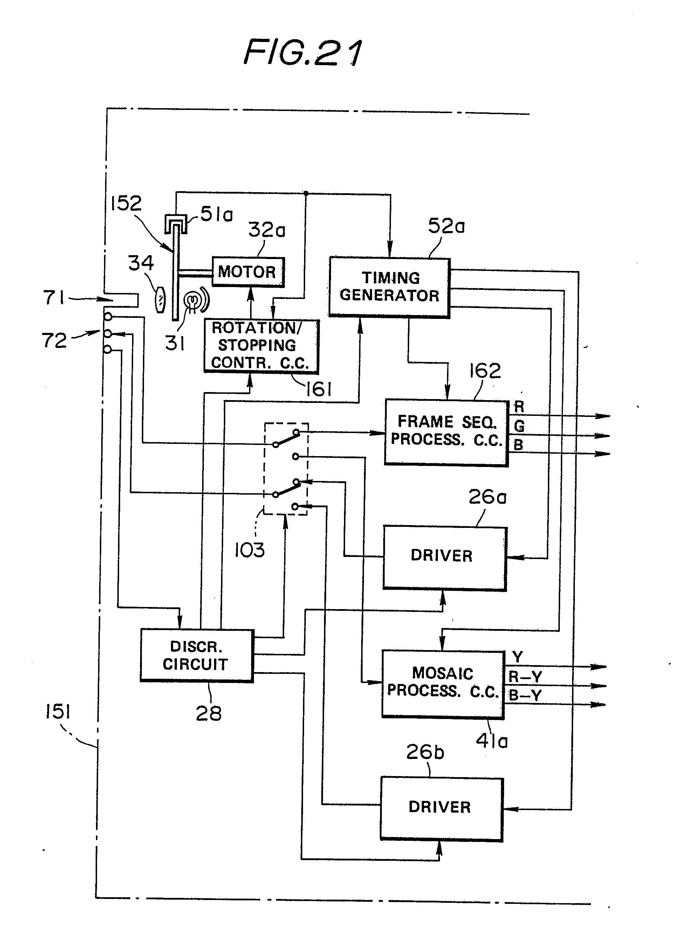

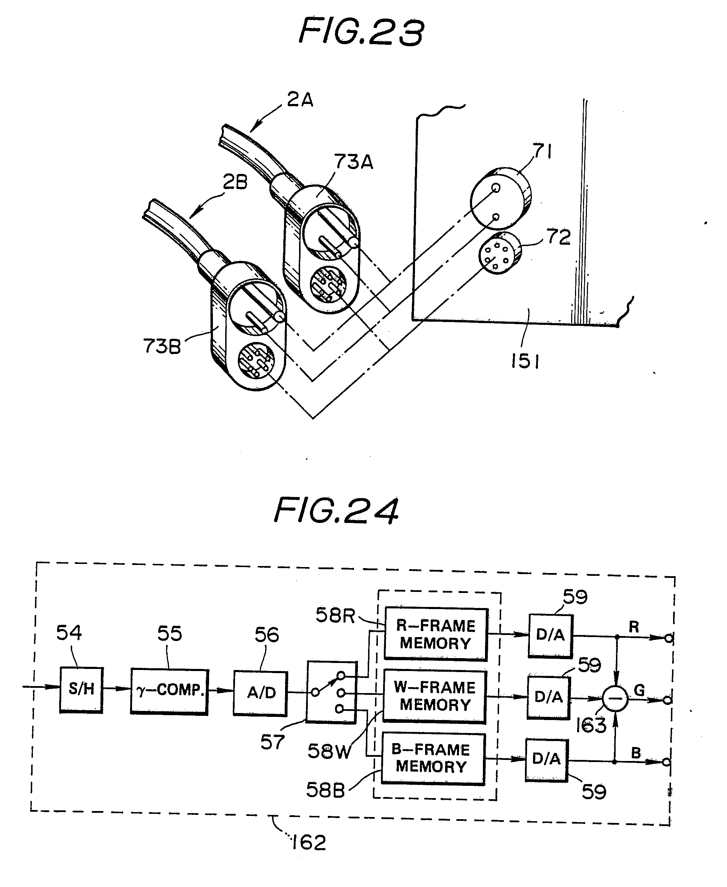

Figure 21 shows an essential part of a video processor apparatus 151 of a seventh embodiment of the present invention, and Figure 22 shows a plan view of the filter disc.

In this embodiment, the sequence of illuminating lights are not of R,G and B as was the case for the embodiment shown in Figure 12, but are of R,W and B.

The rotary filter 152 to be used for the sequential illumination with the illuminating light sequence of R,W and B is provided with fan-shaped windows in a disc-like filter frame 153 as shown in Figure 22. R,W and B colour transmitting filters 154R,154W and 154B are fitted to the respective windows. This W transmitting filter 154W is a filter transmitting R,G and B, and thus it may be an approximately transparent plate to transmit all white light components.

The R,W and B colour transmitting filters 154R,154W and 154B differ in arcuate length so that the illuminating period may be different in response to the photo-sensitive characteristic of the CCD element 18 or 22. In the filter frame 152, separate leading-pulse (detecting) holes 155R,155W and 155B are provided near the respective ends (with respect to the rotating direction) of the R,W and B colour transmitting filters 154R,154W and 154B, so that a timing pulse time, just upon being illuminated with R,W and B, may be detected. The positions of these pulse holes 155R,155W and 155B can be detected by the fact that, when the position aligned with the photosensor 156 arranged opposed to the light emitting device to hold the filter frame 153 is reached, the light of the light emitting device will be received in the form of pulses in the photosensor 156. When this pulse-like light is detected, the detecting signal will be transmitted to the timing generator 52a, and a read-out driving pulse will be applied to the CCD element 18 or 22 through the driver 26a or 26b.

In the filter frame 153, a starting pulse hole 157 is provided, in this example in a position radially adjacent to the leading pulse hole 155R. When this hole reaches a position opposed to the photosensor 158, the photosensor 158 will emit a starting pulse.

Further, in order to detect the position of the W colour transmitting filter 154W, an arcuate slot 159 is formed in an outer peripheral position relative to this colour transmitting filter 154W. The position of the W colour transmitting filter 154W can be determined by detecting this slot 159 with the photosensor 160. The output of this photosensor 160 controls the stopping position of the rotary filter 152. That is to say, in case the motor 32a rotating and driving the rotary filter 152 is not in a rotating driving state, the output of the photosensor 160 is input into a rotation/stop controlling apparatus 161 to control the stopping position of the rotary filter 152 so that the stopping position of the rotary filter 152 may be the position aligned with the photosensor 160. In this stopping position state, the illuminating light of the light source lamp 31 passes through the W colour transmitting filter 154W and can feed a white illuminating light via the light source connector socket 71. When a fibre scope is connected to the connector socket 71, but nothing is connected to the connector socket 72, or when nothing is connected to the connector sockets 71 and 72 (both of which states can be detected by the discriminating circuit sensing the high impedance state), or when a mosaic type scope is connected, then this white illuminating state will be held.

On the other hand, when a frame sequential type scope is connected, the connection will be sensed by the discriminating circuit 28, a motor 32a energised, and driving instruction signals fed to the rotation/stop controlling circuit 161, to rotate and drive the motor 32a in a frame sequential illuminating operational state.

In this embodiment, the light source connector socket 71 of the apparatus 151 can be used in common for the white colour and frame sequence operations. The signal connector socket can be used in common for the frame sequential type and mosaic type as shown, for example, in Figure 23. Two electronic scopes 2A and 2B are shown in Figure 23. The other scopes 2C,2C and 2E can be connected when desired.

In this embodiment, as the illuminating light sequence is not R,G and B, the frame sequential process circuit 162 is as shown, for example, in Figure 24. That is to say, the G frame memory 58G shown in the processor circuit 41a in Figure 7 is replaced with the W frame memory 58W, the memory contents thus being different, but in fact, the same frame memory can be used in the actual hardware. The W colour signal read out of the W frame memory 58W and forming an analogue signal from the accociated D/A-converter 59 is fed into a substractor stage 163 to have the R colour signal and B colour signal deducted and so produce the required G colour signal. The other elements are the same as in the processor circuit 41a shown in Figure 7.

The other formations of the apparatus 151 shown in Figure 21 are the same as those shown in Figure 12.

According to this embodiment, both frame sequential type and mosaic type commonly use the light source and can be easily used when merely a scope is connected. Also, there is no need to provide any supplementary equipment, such as means for moving the light source or rotary filter. The cost can be reduced and the size can be made small.

In the above-mentioned embodiment, the light source connector means and signal connector means are commonly used, but separate signal connector means could be utilised if so desired.

In the above-mentioned embodiment, the frame sequential illumination is made with R,W and G, but is not limited to this. The illumintion can be made, for example, with R,G and W; W,G and B; Cy(cyanine),Ye(yellow) and W; Cy,W and Mg(magenta); W,Ye and Mg, etc.

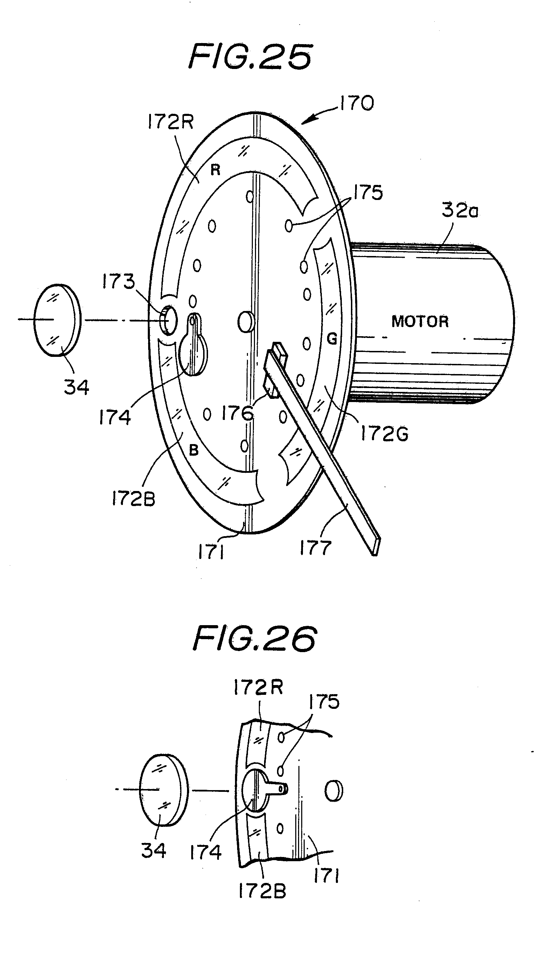

Figure 25 shows the periphery of a rotary filter 170 used in the eighth embodiment of the present invention.

In this embodiment, R,G and B colour transmitting filters 172R,172G and 172B are provided in a filter frame 171. A white illuminating aperture 173 is provided in a light intercepting part, for example, between the R and B colour transmitting filters 172R and 172B, and can intercept the light with a light intercepting plate 174 rotatably fitted with a positionin the course of a line segment connecting the hole 173 and the centre as a pivotal point.

Thus, when the filter frame 171 is rotated by the motor 32a, the light intercepting plate 174 is moved to cover the aperture by the centrifugal force, as shown in Figure 26. The direction connecting the centre positionof the disc-like light intercepting part and the pivotal point will coincide with the radial direction in this state so the aperture 173 will be covered by the light intercepting plate and ordinary R,G and B frame sequential illumination will be provided.

On the other hand, when the disc is stationary, no centrifugal force is generated and therefore the light intercepting plate 174 will retract and uncover the hole 173 due to the gravity, as shown in Figure 25.

The filter frame 171 is position controlled so that, when stopped, the hole 173 is aligned with the optical axis connecting the light source lamp and lens 34. For controlling the position or for detecting the timing of reading out the CCD signal in the case of the R,G and B frame sequence, a plurality of holes 175 are provided around the filter frame 171, and a light emitting device and photosensor 176 are arranged on both sides of the plate surface of the filter frame 171 to form a position detecting rotary encoder in known manner. In Figure 25, the photosensor 176 is fitted to the tip of the sensor fitting plate 177.

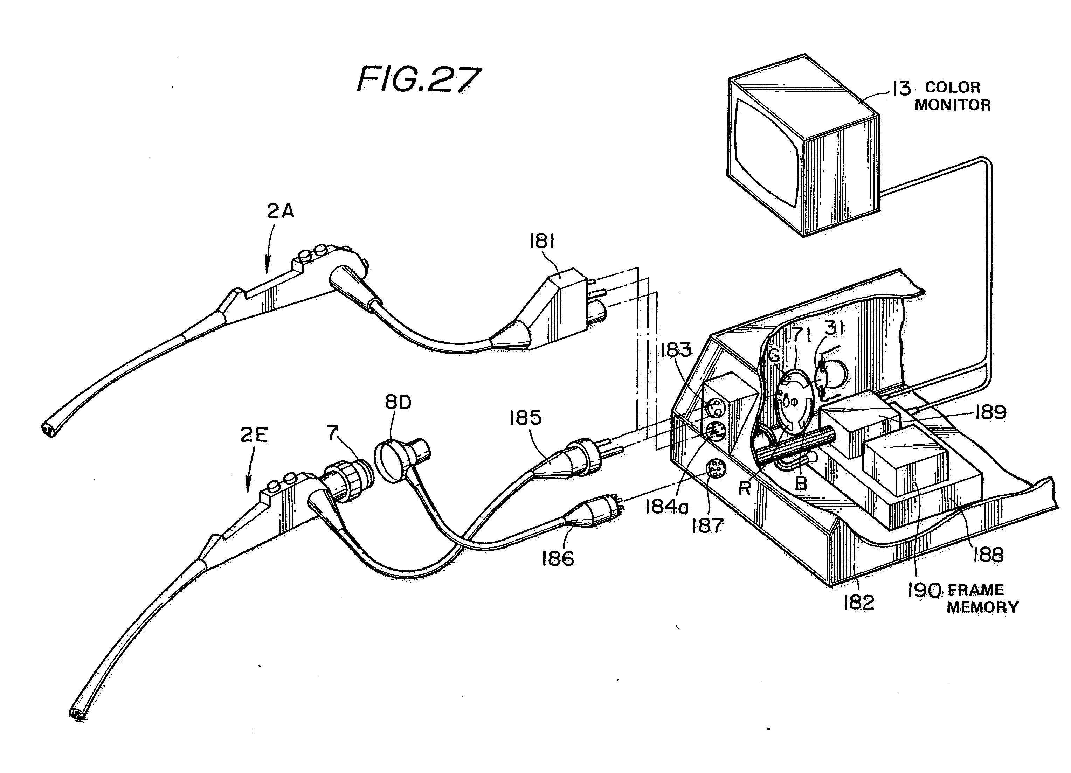

The system of operation of this embodiment is as that of the structure shown in Figure 27 for example.

Figure 27 shows, for example, a frame sequential type electronic scope 2A, a fibre scope 2E and a mosaic type TV camera 8D adapted to be connectible to this fibre scope 2E.

The connector 181 of the frame sequential type electronic scope 2A has a combined light source connector and signal connector, made integral in a manner to permit it to be connected to a light source connector socket 183 and frame sequential type connector socket 184a.

On the other hand, the connector 185 of the fibre scope 2E can be connected to the light source connector socket 183 to make observations by the naked eye. If a mosaic type TV camera 8D is fitted against the eyepiece 7, this forms a scope fitted with a mosaic type TV camera, and the signal connector 186 of this mosaic type TV camera 8D need only be connected to the mosaic type signal connector socket 187 to complete the operational system.

Though not shown in Figure 27, the mosaic type electronic scope 2B can also be used. The fibre scope 2E can also be used with the frame sequential type TV camera 8C connected.

The formation within the apparatus 182 is substantially the same as that of a combination of Figures 19 and 21. (The light source 142 and its controlling circuit system shown in Figure 19 are replaced by the light source of Figure 21, and the filter is replaced with the rotary filter shown in Figure 25 so that R,G and B frame sequential lights or a white light may be output). These are arranged as shown in Figure 27.

For example, the frame sequential type video processor stage is contained within a box-like housing 188, and a housing 189 containing a mosaic type video processor stage is arranged on the upper surface of the housing 188. A frame memory 190 forming a frame sequential type video processor stage is also arranged on the upper surface of the housing 188.

A colour monitor 13 is connected via a signal cable to the signal output ends of both housings 188 and 189.

The filter frame 171 forming part of the rotary filter 170, and the associated light source lamp 31 are arranged behind the light source connector socket 183.

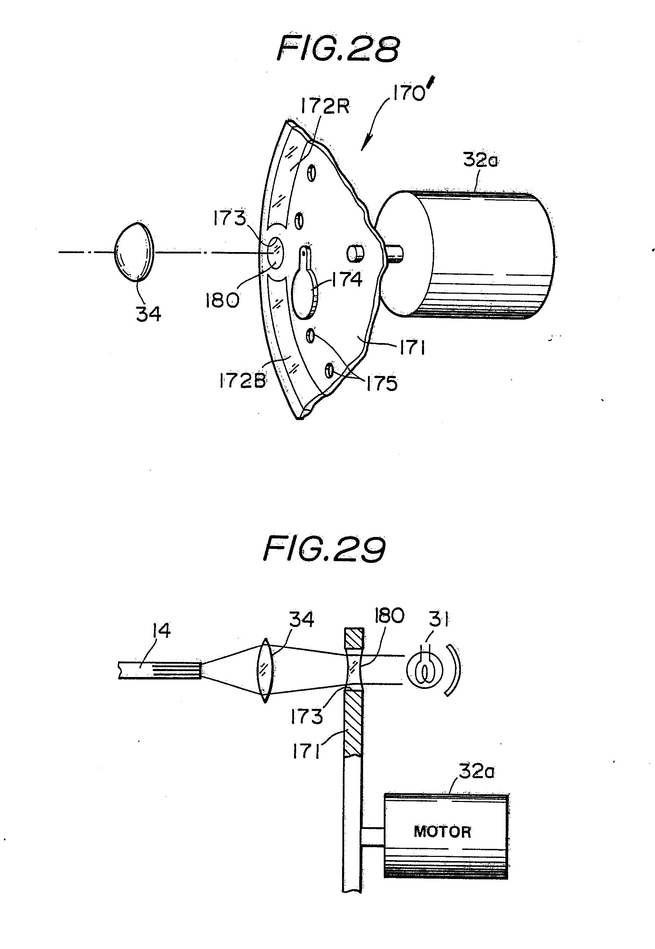

Figure 28 shows a rotary filter 170' of a modification of the seventh embodiment.

In this rotary filter 170', a concave lens 180 is fitted in the aperture 173 of the filter frame 171 shown in Figure 25, and a cross-section of this light source and the aperture 173 is shown in Figure 29.

The concave lens 180 projects the illuminating light in condensed and defocussed form onto the light guide fibre end-face in the case of illumination with a white light, the defocussing ensuring that the light guide fibres do not overheat. When the concave lens 180 is interposed, that is, in cases where the light is passed through the filter, it will be focussed on the light guide fibre end-face. In such a case, the light will be reduced by the filter and therefore the light guide fibre end-face will not be substantially heated. By moving the lens 34 or light source lamp 31 in the optical axis direction on rails provided for the purpose (not shown), the light can be defocussed in the case of illumination with white light, and, by a resetting when frame sequential operation is required, the light can be refocussed.

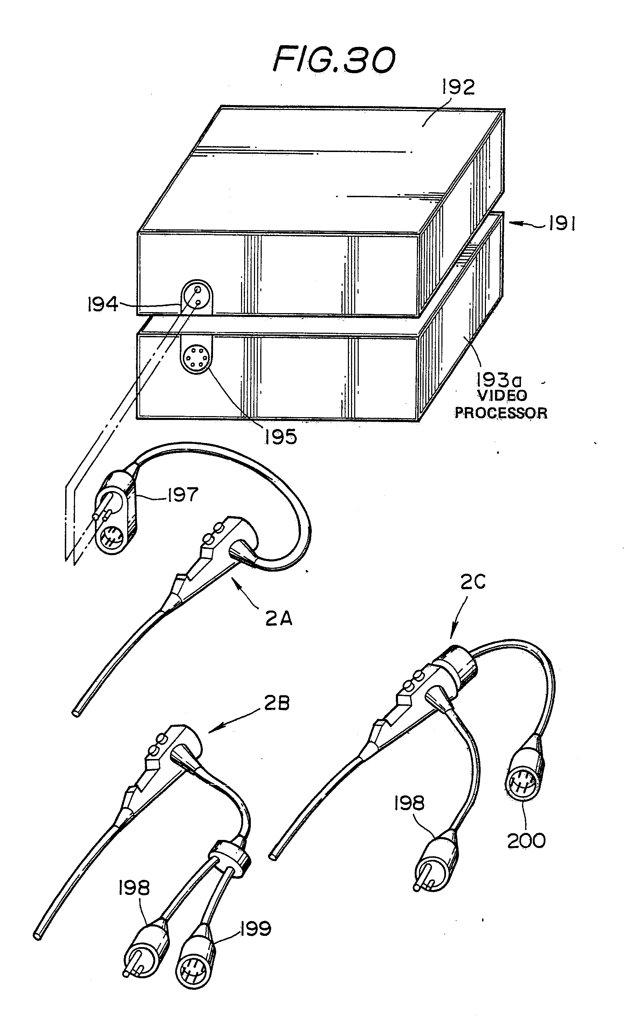

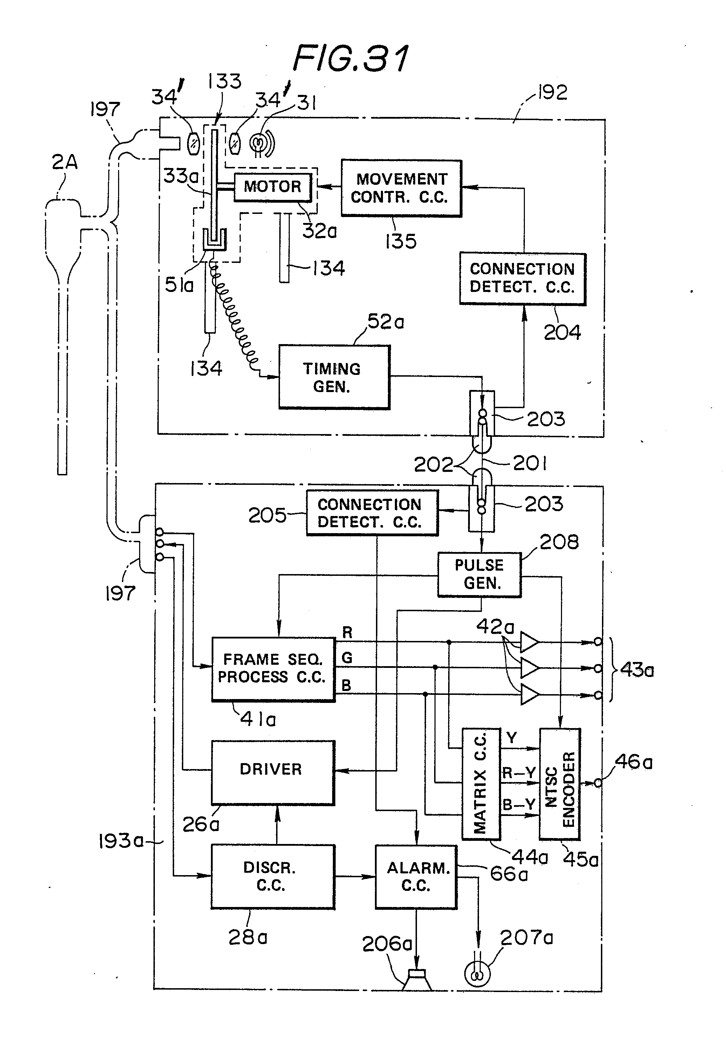

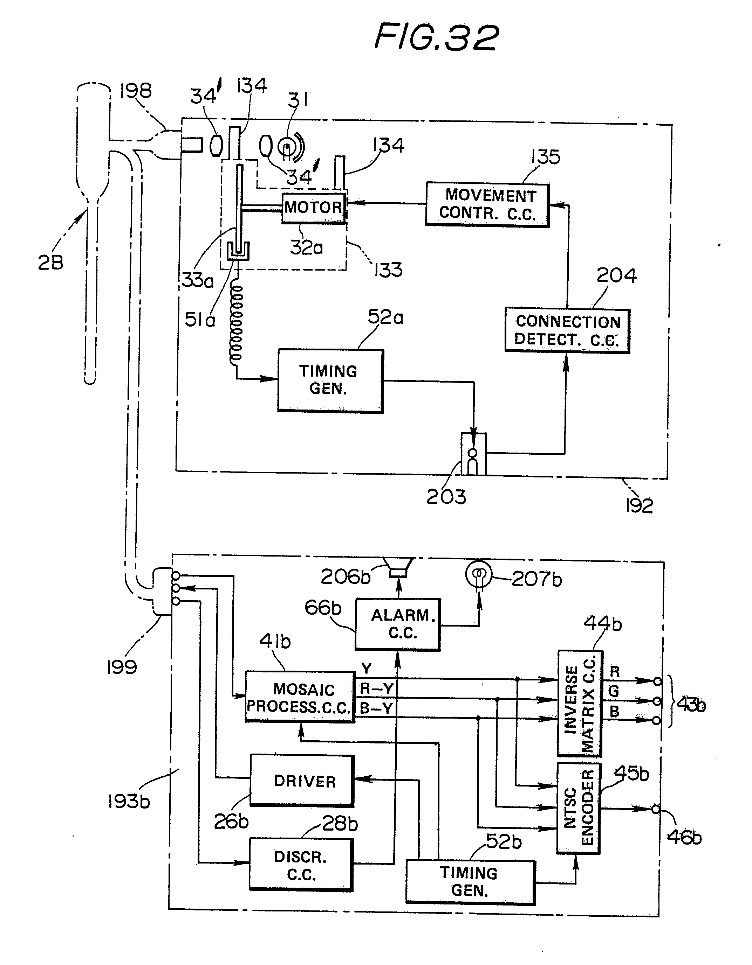

Figure 30 shows the contour of the ninth embodiment of the present invention, whilst Figure 31 shows the system used in a frame sequential type scope as assembled, and Figure 32 shows the system used in a mosaic type scope as assembled in this ninth embodiment. An imaging apparatus 191 is formed of a separate and commonly used light source 192, and a frame sequential type video processor stage 193a, which is as shown in Figure 31 or a mosaic type video processor stage 193b as shown in Figure 32. As shown in Figure 30, a light source connector socket 194 is provided at the lower edge of the front surface of the light source 192. On the other hand, a signal connector socket 195 is provided at the upper edge of the front surface of the video processor stage 193a or 193b. These connector sockets 194 and 195 will be adjacent to each other, above and below the junction line, when the light source 192 is overlaid on the upper surface of the video processor stage 193a or 193b (one of the video processor stages 193a is shown in Figure 30).

In the frame sequential type electronic scope 2A, its connector 197 combines the light source connector and signal connector as an integral unit. As shown in Figure 30, when the light source 192 is fitted to overlap the video processor stage 193a, both connector parts can be connected to the respective connector sockets 194 and 195.

On the other hand, for example, if the mosaic type electronic scope 2B is used, its connector is separated into a light source connector 198 and a signal connector 199, which can be connected respectively to the connector sockets 194 and 195. If the fibre scope 2C is fitted with a frame sequential type TV camera, the light source connector 198 and signal connector 200 can be connected respectively to the connector sockets 194 and 195.

The light source 192 is of a formation similar to that of the light source in Figures 16 or 18. The lens 34 in Figure 16 is replaced by two lenses 34' in this embodiment.

Internal details of the light source and a sequential video processor stage are shown in Figure 31.

The light source 192 is provided with a connector socket 203 connecting one of connectors 202 of a cable 201 to feed a timing pulse of the timing generator 52a to the frame sequential type video processor stage 193a, and the frame sequential type video processor stage 193a is provided with such a link to the light source in the same manner.

The light source 192 is provided with a connection sensing circuit 204 for sensing whether or not the connector 202 of the signal cable 201 is connected to the connector socket 203. When the cable 201 is connected, as shown in Figure 31, a movement instructing signal will be emitted to the movement controlling circuit 135 by the output of this circuit 24, the rotary filter 133 will be moved along the rails 134 to interpose the rotary filter 333a in the illuminating light path, and a frame sequential illumination will be produced.

A connection sensing circuit 205 for sensing whether or not the connector 202 of the signal cable 201 is connected to the connector socket 203 is provided within the frame sequential video processor stage 193a. The output of this sensing circuit 205 is input into the warning circuit 66a. When this warning circuit 66a senses from the discriminating circuit 28a that the frame sequential scope 2A or 2C is connected, if a sensing signal showing that the cable 201 is not connected is received from the connection sensing circuit 205, it will be indicated by a warning buzzer 206a and warning light 207a that the cable 201 is not connected. Also, it will be indicated if the signal connector 199 of the mosaic type scope 2B or 2D is connected to the signal connector socket 195.

Through the cable 201, the timing pulse from the light source 192 emits a control signal to the driver or the like through the pulse generator 208 within the video processor stage 193. The other elements are the same as those shown in Figure 16.

The formation of the mosaic type video processor stage 193b shown in Figure 32 is similar to that shown in Figure 16.

The video processor stage 193b is provided with a warning circuit 66b operated by the output of the discriminating circuit 28b. If the signal connector of the frame sequential type scope 2A or 2C is connected to the mosaic type signal connector socket 195, this warning circuit 66b will sense the mis-connection, and it will be indicated by the buzzer 206b or warning light 207b. The other elements are of the same formation as that shown in Figure 16.

In the event that the mosaic type scope 2B or 2D, or the fibre scope 2E is connected, the rotary filter 133 will not be moved on the rails 134, and therefore the white light of the light source lamp 31 will be condensed and radiated to the connector 198 through the two lenses 34'.

Figure 31 shows the frame sequential type electronic scope 2B as connected, but its connector 197 is shown as separated parts for the sake of clarity and convenience.

In this embodiment, even if the connector is integral as in the case of the frame sequential type scope 2A, or is separated as in the case of the mosaic type scope 2B, it can in actual fact be connected.

In Figure 30, the connector 197 of the frame sequential type electronic scope 2A is made integral for the light source and for the signal, but it may be formed separately as in the case of the mosaic type electronic scope 2B. In just the same way, the connectors 198 and 199 of the mosaic type electronic scope 2B may be made integral, if so desired.

The connection sensing circuits 204 and 205 are not always necessary. In the described ninth embodiment, the rotary filter 133 is made movable, but equally the lamp 31 and connector socket 195 may be made movable, and a fixed axis provided for the filter if so desired.

A signal for increasing or decreasing the light output of the lamp 31 may be sent through a signal line (not illustrated) to the light source 192 from the video processor stages 193a and 193b, to automatically adjust the light.

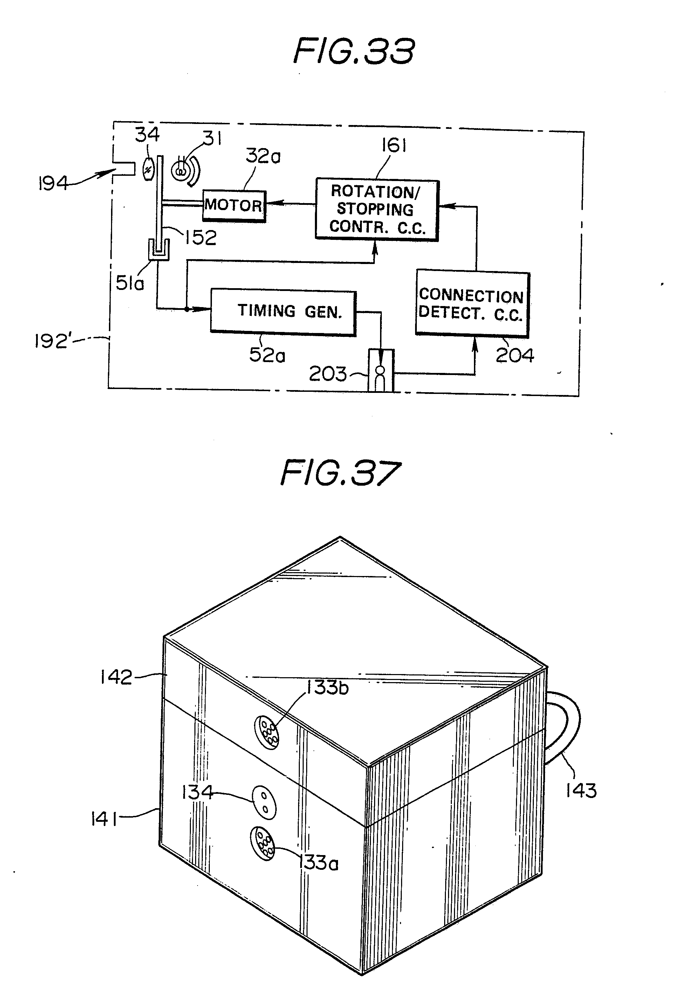

Figure 33 shows a modification of the light source 192 in the ninth embodiment, to form a light source 192' in which the movable structure of the rotary filter 152 shown in Figure 22 is used instead of the rotary filter 133 of Figue 31, the rotation/stop control being effected by the rotation/stop controlling circuit 161 (Figure 21). In this case, the field sequential type processor circuit 162 shown in Figure 24 is used. This embodiment has substantially the same function as that of the initially-described ninth embodiment.

Instead of the rotary filter 152, the rotary filter 170 shown in Figure 25 may be used. In this case, the frame sequential type processor circuit 41a shown in Figure 31 can be used.

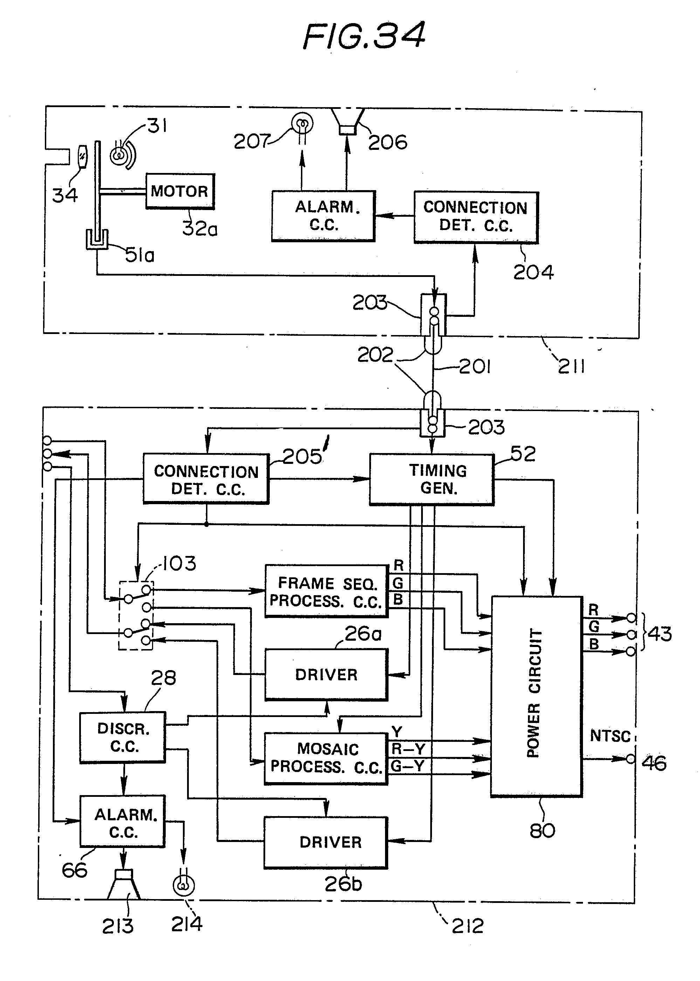

Figure 34 shows the tenth embodiment of the present invention.

In this embodiment, the light source 211 is used which is not provided with the timing generator 452a in the light source 192' shown in Figure 33, but shares a common timing generator 52 within a common video processor stage 212. For frame sequential illumination, unless the cable 201 is connected by the connection sensing circuit 204, it will be indicated by sounding the buzzer 206 or by lighting the lamp 207.

The video processor stage 212 is provided with a connection sensing circuit 205' (the same as in the ninth embodiment), a buzzer 213 driven by the warning circuit 66, and a warning light 214 in addition to those (206 and 207) shown in the unit 211. This connection sensing circuit 205' has the same function as of the connection sensing circuit 205 shown in Figure 31 for the ninth embodiment.

The buzzer 213 and warning light 214 are operated by the output of the discriminating circuit 28 and have the same function as on the operation by both discriminating circuits 28a and 28b of the ninth embodiment.

The other elements are of the same formation as in the ninth embodiment.

According to this tenth embodiment, as the video processor part 212 is commonly used, the number of units forming the entire system can be reduced, and apparatus movement made more convenient. As at least a part of the signal processing system is commonly used, the number of component parts can be reduced and the cost made low.

Instead of the output circuit 80 provided with the signal converting function as the ninth embodiment, the output circuit 113 shown in Figure 14,or the one shown in Figure 15, may be used.

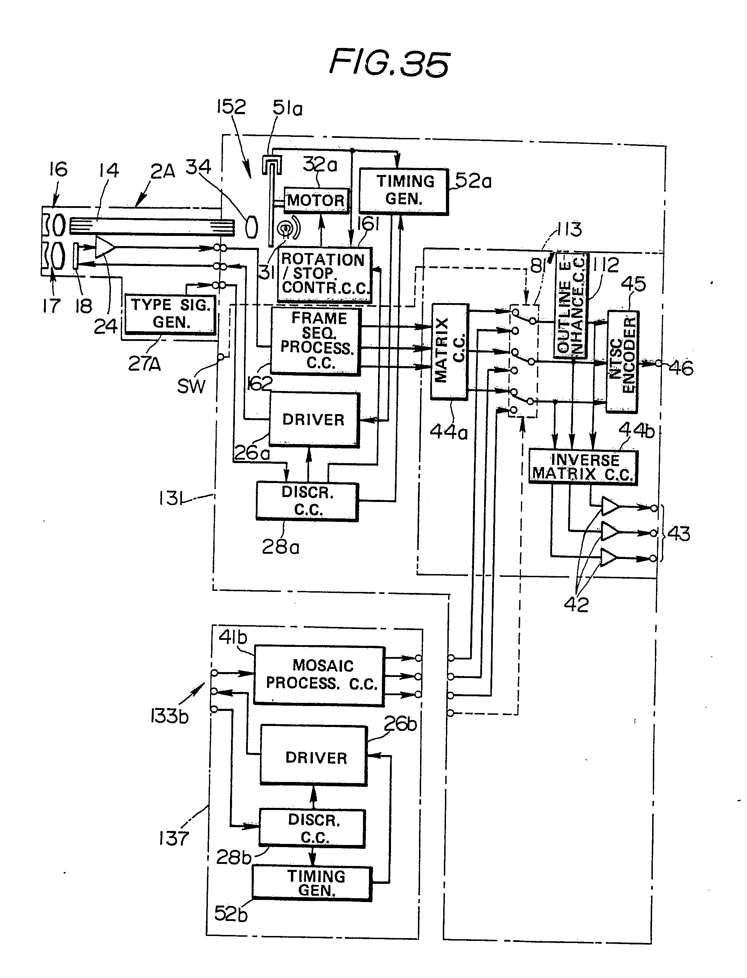

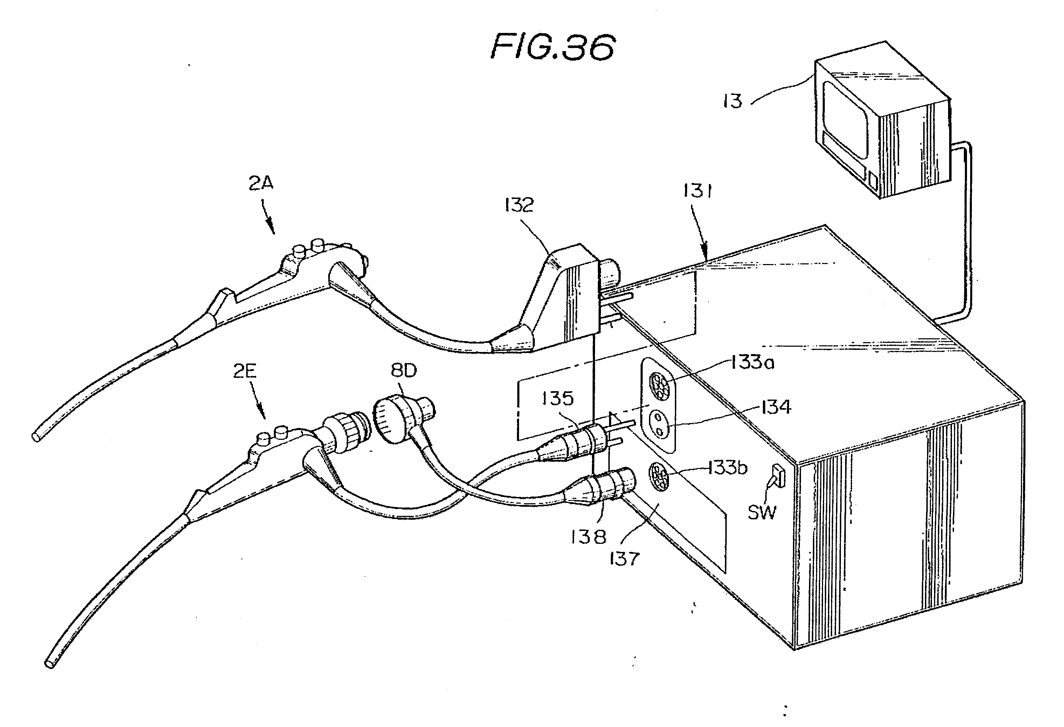

Figure 35 shows the internal construction of the eleventh embodiment of the present invention, and Figure 36 shows the contour.

The video processor unit 131 in Figure 36 is provided with a light source connector socket 134 used in common with a frame sequential type signal connector socket 133a so that the connector 132 of the frame sequential type electronic scope 2A may be connected. The image is colour-displayed by a colour monitor 13 (as shown in Figures 1, 27 or 48).

The connector (not illustrated) of the fibre scope 2C fitted with the field sequential type TV camera can be used as connected to the connector sockets 133a and 134.

The fibre scope 2E would have its connector 135 connected to the light source connector socket 134 so that a naked eye observation may be made.

The light source inside the light source connector socket 134 can normally output a white colour light. When the rotary filter is rotated, a frame sequential illumination will be emitted.

When the frame sequential type connector socket is connected to the frame sequential type signal connector socket 133a, the rotary filter will be rotated by the type signal output to provide a frame sequential illumination.

A recess is provided in the lower side of the front surface of the video processor unit 131 so that the mosaic type pre-processor unit 137 may be plugged in and fitted. A mosaic type signal connector socket 133b is provided on the front surface of this mosaic type pre-processor unit 137. The signal connector 138 of the mosaic type TV camera 8D or the signal connector (not illustrated) of the mosaic type electronic scope 2B can be connected to this connector receptacle 133b.

As shown in Figure 35, within the video processor unit 131, the same light source as shown in Figure 21 is contained, and also the frame sequential processor stage 162 is contained. This frame sequential type processor stage is substantially the same as that selected when the switch 103 of the seventh embodiment is switched to the frame sequential side in the processor stage shown in Figure 21, and, further on its output side, is made an output circuit 113 provided with a funciton of processing the outline enhancing signal, as shown in Figure 14.

The switching switch 81′ within the output circuit 113 provided with this signal processing means will be switched if a mosaic type pre-processor unit 137 is plugged in.

According to this embodiment, even if the mosaic type pre-processor unit 137 is to be acquired (bought) later, when required, the mosaic type scope can be used and the function of the apparatus can be economically expanded.

If the mosaic type pre-processor unit 137 is plugged in, the frame sequential type and mosaic type can still be switched and used as a selected switch SW is provided, for example, on the front face of the processor unit 131, and the switching of this switching switch 81′ can be controlled with this switch SW.

In the tenth embodiment, the plug-in unit can be fitted on the front face as indicated in Figure 36. However, the mosaic type video processor unit 113, or a part of it, is fitted into a tapering slot provided in the rear face ( or as proves most convenient for manufacture and use), so as to make the whole assembly available for scopes of either the frame sequential type or mosaic type by simple actution of the switch SW or the like.

Also, as shown in Figure 37, if a mosaic type video processor stage 142 is fitted on the upper face of an apparatus body 141 provided with a signal processing means for the scope of the frame sequential type, and a signal cable 143 from this mosaic type video processor 142 is connected to the connector socket of the video processor unit 141, the apparatus 141 may be used for scopes of either system.

On the front face of the apparatus 141, connector sockets 133a and 134 are provided and, on the mosaic type video processor stage 142, a connector socket 133b is provided.

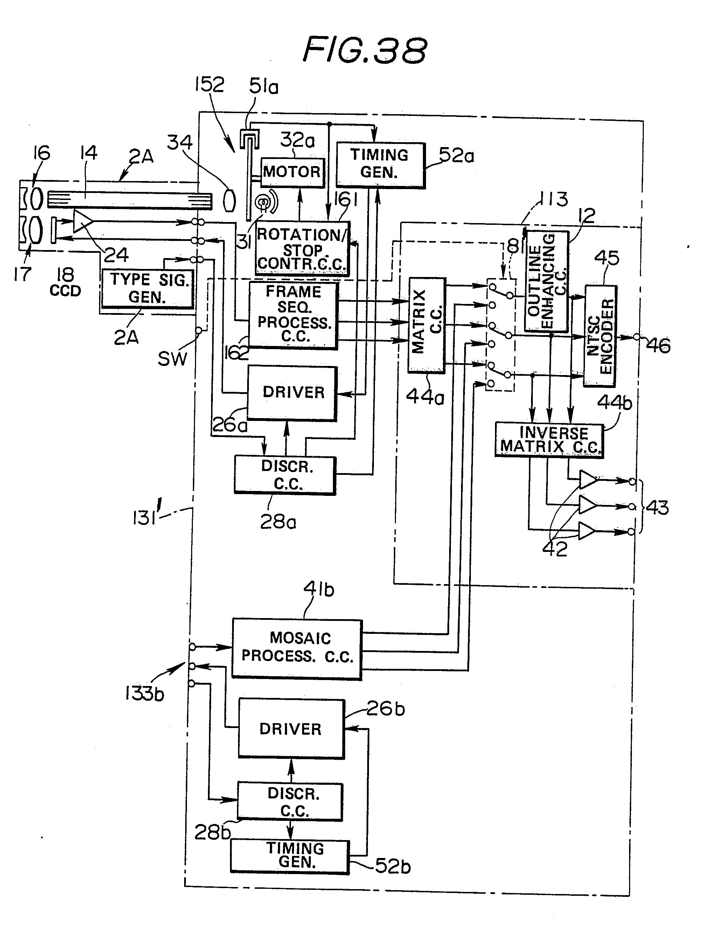

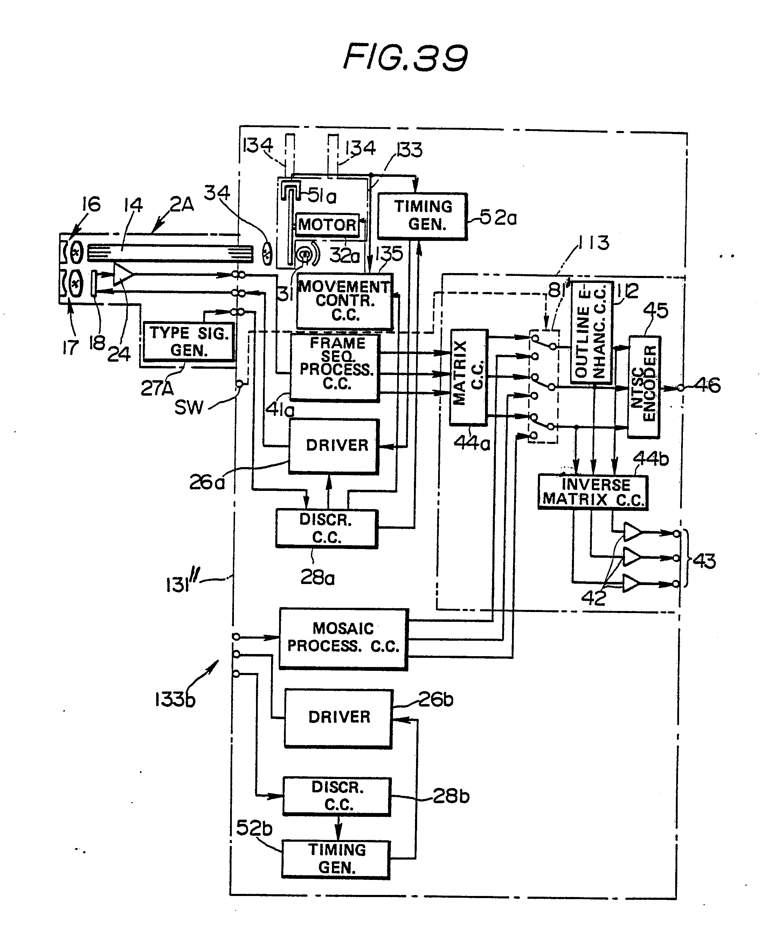

The apparatus having the format shown in Figure 35 and Figure 36 may be made as an integrally-combined video processor stage 131′ including the circuitry of the unit 137 shown in Figure 36, and such an arrangement is shown in Figure 38. In the video processor stage 131′ shown in Figure 38, the light source shown may be replaced by that shown in Figure 18, for example, to make an arrangement 131″ shown in Figure 39. In this case, the frame sequential type processor stage 41a shown in Figure 2 is used, for example. In Figures 38 and 39, signals other than for enhancing the outline may be processed.

Further, in Figure 35, both drivers 26a and 26b and both discriminating circuits 28a and 28b could be commonly used. The light source shown in Figure 35 may be replaced with one of another format.

In the apparatus shown in Figure 35, the frame sequential video processor unit 131 has a mosaic type unit 137 (Figure 36) plugged in and fitted to be used. Alternatively, a frame sequential type unit can be fitted as an addition to a unit of the mosaic type.

The number of pixels of the CCD element 22 of the TV camera 8C or 8D connected to the fibre scope 2E may be made larger tn the number of pixels of CCD element 18 of the electronic scope 2A or 2B, so that the resolution may be improved. If the number of pixels of the TV camera 8C or 8D is thus made larger, a signal processing circuit corresponding to the number of pixels in the case of the TV camera 8C or 8D may be provided.

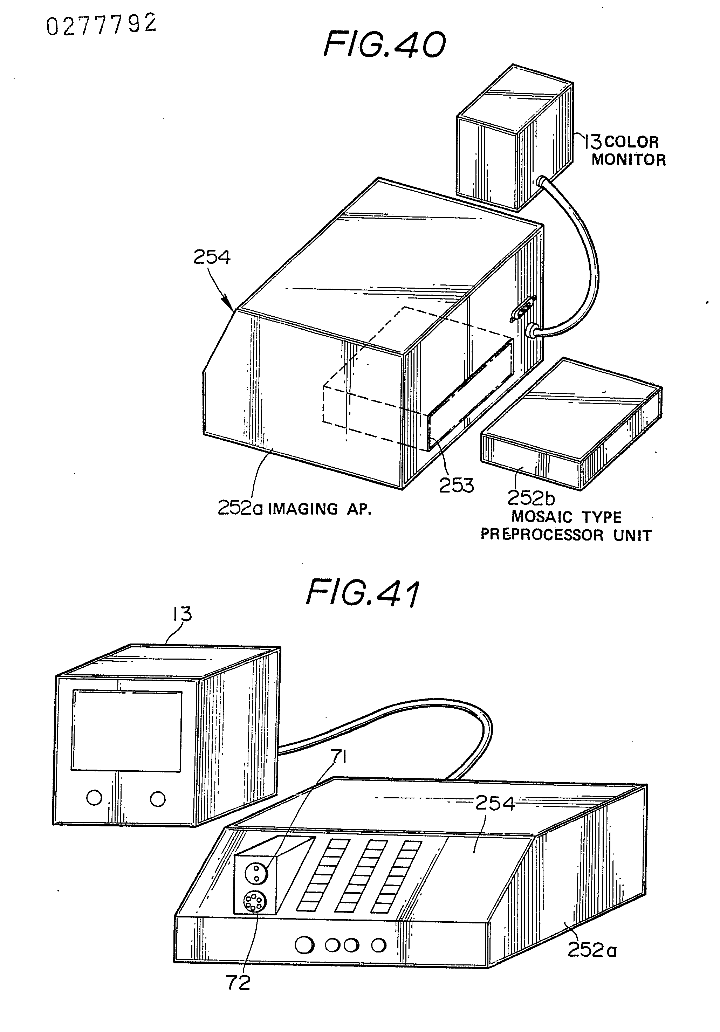

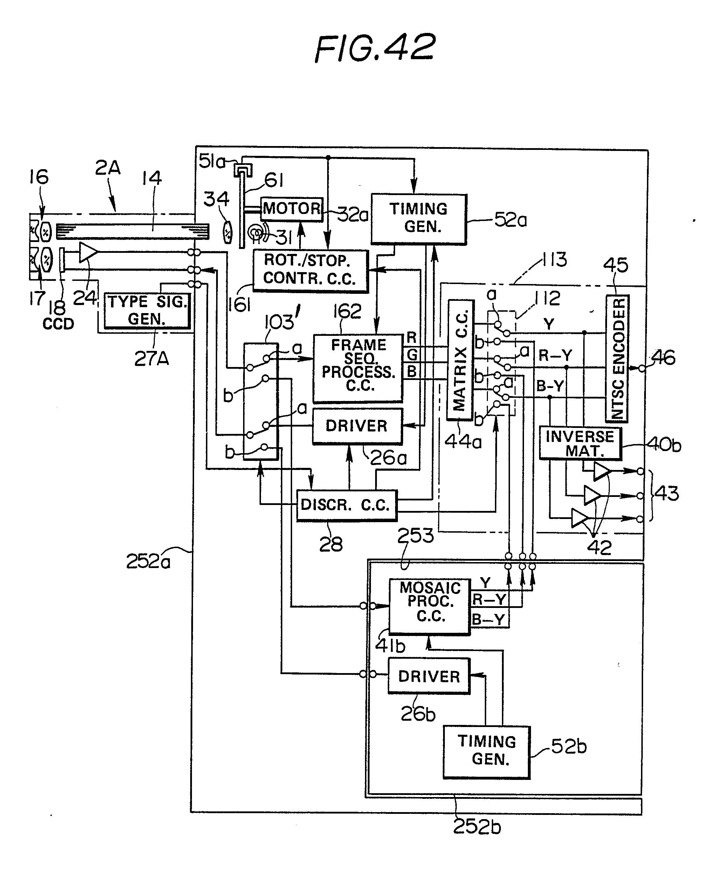

In Figure 36, the mosaic type pre-processor stage 137 can be fitted to the front face of the unit 131. As shown in Figure 40, the mosaic type pre-processor unit 252b may be inserted into a fitting port 253 provided on the rear of the apparatus 252a provided with a frame sequential signal processing means as shown in Figure 42.

As shown in Figure 41, on the front face of the apparatus 252a, a panel 254 is provided with a light source connector socket 71 and signal connector socket 72.

The circuit formation of the apparatus 252a and mosaic type pre-processor unit 252b is as shown in Figure 42. This circuit formation is a combination of that shown in Figure 21 and that shown in Figure 35.

When the apparatus 252a is fitted with the mosaic type pre-processor unit (mosaic type unit) 252b, the imaging apparatus can be used for the mosaic type scopes 2B and 2D. That is to say, in this apparatus 252a, th einput signal switch 103′ is normally held ON making contact to the b side, and the switch 112 within the output circuit 113 is also held ON making contact to the b side. Therefore, when the mosaic type scope 2B or 2D is connected, a driving signal will be applied to the CCD 19 through the switch 103′ from the driver 26b and the signal read out by this application will be input into the mosaic type processor circuit 41b through the switch

103'.

In this case, the discriminating circuit 28 will emit a stopping control signal to the rotation/stop circuit 161 and the rotary filter 61 will be held in the stopped state. Therefore, in this case, a white colour light will be fed out.

When a frame sequential type scope 2A or 2C is connected, the frame sequential type will be identified by the discriminating circuit 28 and the switch 103' will be switched over to make contact on the a side. The switch 112 will also be switched over to contact the a side, and so select the matrix 44a.

The discriminating circuit 28 applies a rotating control signal to the rotation/stop control circuit 161, rotates the rotary filter 61 fitted to the motor 32a, and emits a frame sequential light sequence.

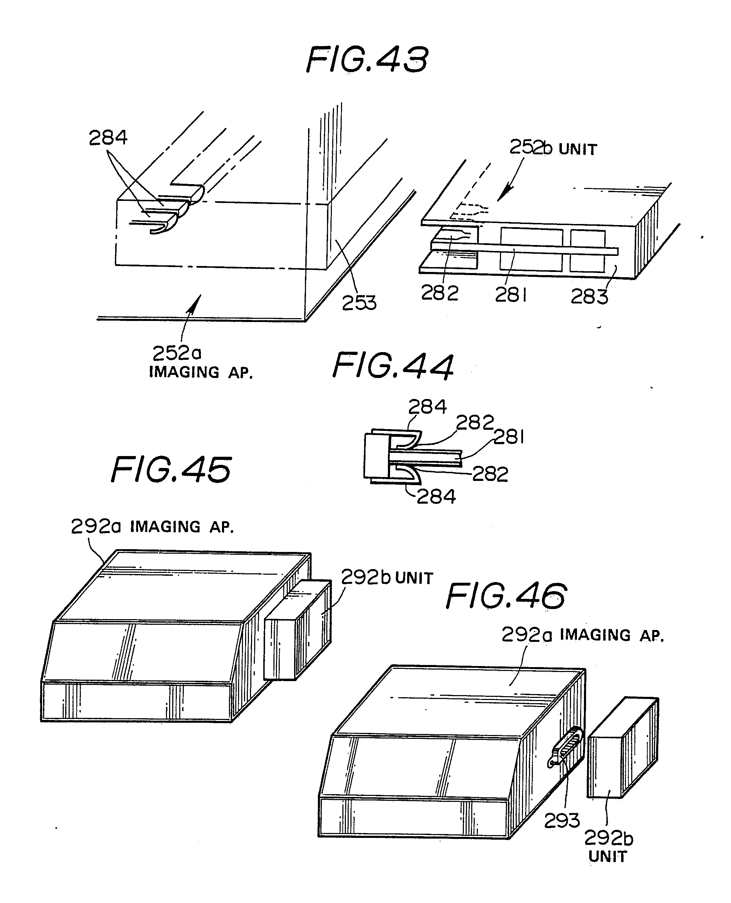

The structures of the fitting port 252 and of the connecting part of the unit 252b inserted into this fitting port 253 are shown in Figure 43.

The end part of a substrate 281 is present at the edge of the removable mosaic type unit 252b, and is provide with connecting lands 282. Both upper and lower surfaces and the other end of this substrate 281 are covered by a housing 283.

On the other hand, the fitting port 253 of the apparatus 252a is provided along its innermost face with connecting tags 284 (only the upper side tags are shown in Figure 43), each of which tag holds the associated land 282 of the inserted unit 252b from both the upper and lower side (as shown in Figure 44), to hold the electric connection firmly, and secure the fitted state.

When this unit 252b is fitted, the unit 252b may be flush with the rear surface of the imaging apparatus 252a, may somewhat project, or may be somewhat retracted.

According to the thus formed twelfth embodiment, in the case of first buying it (assuming for example that it cannot be bought en bloc due to the budget, for example), the frame sequential type scope apparatus, that is the frame sequential type scope 2A or 2C (or both), frame sequential type imaging apparatus 252a, and monitor 13 can be bought and used.

When the mosaic type unit 252b is bought, any scope can be used. This unit 252b can be simply fitted by being inserted into the fitting port 253.

Figure 45 shows the thirteenth embodiment of the present invention, in which a mosaic type pre-processor unit 292b can be fitted to the side of a frame sequential type apparatus 292a.

In this case, as shown in Figure 46, a connector socket 293 is provided on the side of the apparatus 292a. On the other hand, a connector to be removably fitted to the connector socket 293 is provided on one surface of the unit 292b. By fitting this connector to the connector socket 293, both units become electrically connected and can be held as fitted.

The parts other than the connecting parts are the same as in the twelfth embodiment.

The operation and effect of this embodiment are substantially the same as in the twelfth embodiment. The contour of the unit 292b may conform to the side shape of the apparatus 292a, or may be fitted in any convenient manner.

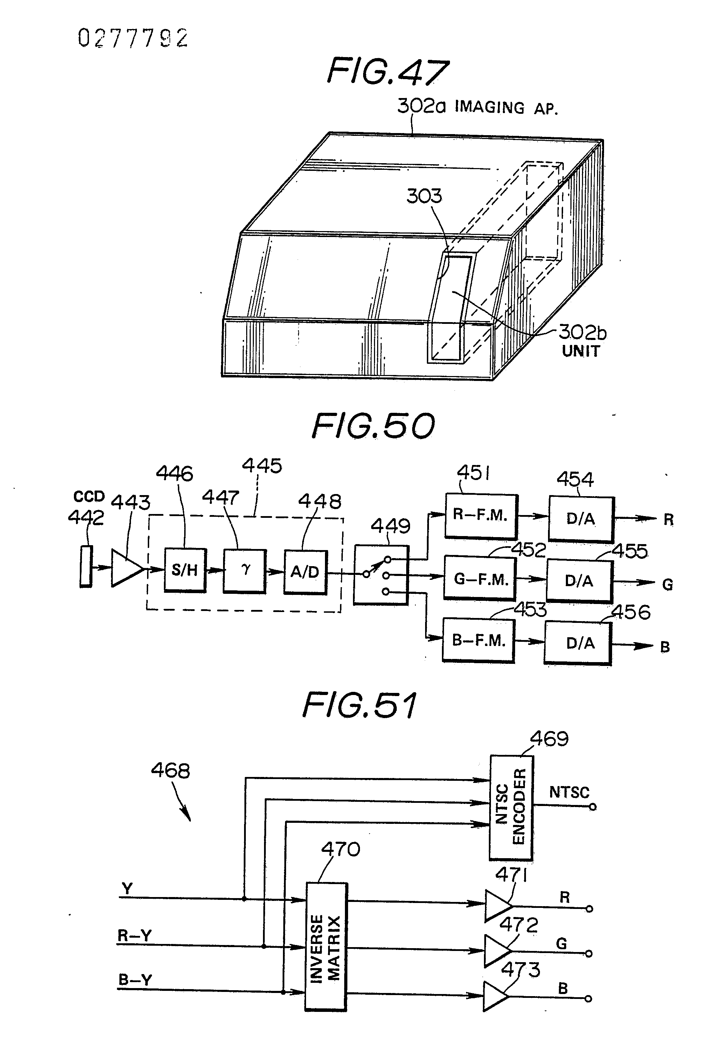

Figure 47 shows a modification of the thirteenth embodiment of the present invention, in which the unit 252b is not fitted to a side or rear face of the apparatus 252 but, in this modification, a mosaic type unit 302b is inserted into a fitting port 303 provided in a position, for example, near one side of the front face of an apparatus 302a.

With this modification, where the unit 302b is fitted into the front face of the apparatus 302, the rear face of the inserted unit (which remains exposed at the front panel of the apparatus 302a) should be flush with the surface of the panel so as to maintain an attractive appearance.

The operation and effect of this modification are substantially the same as for the previously-described embodiment.

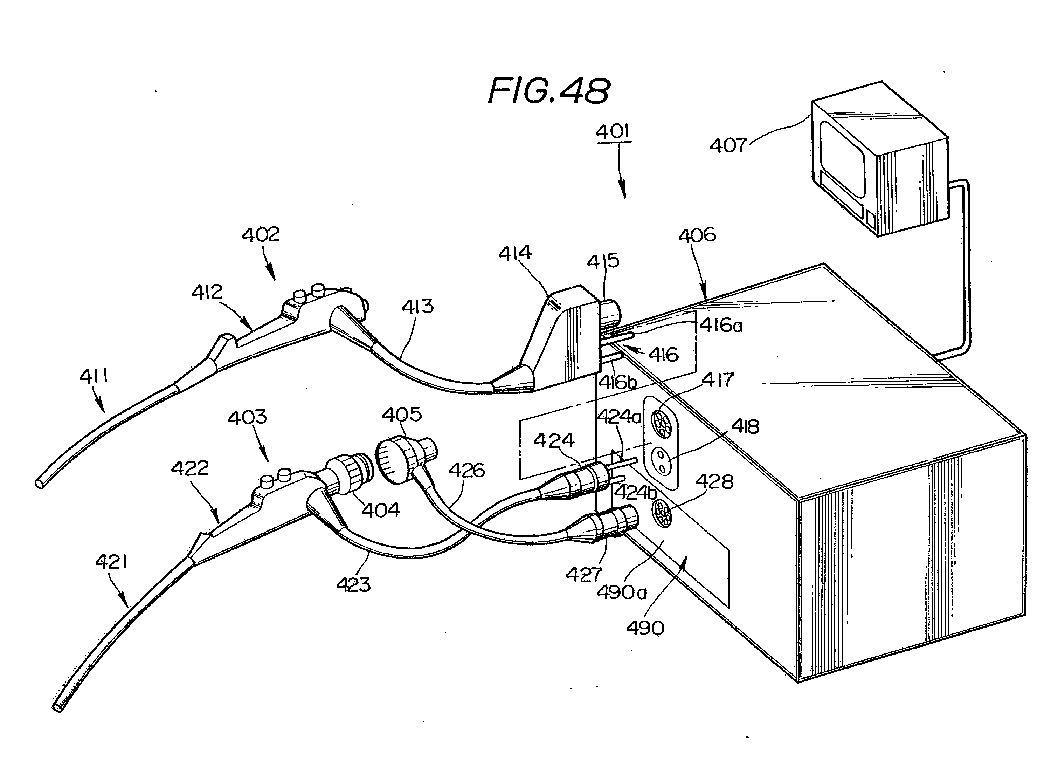

Figure 48 shows the fourteenth embodiment of the present invention, in which an endoscope system 401 comprises a frame sequential type electronic endoscope 402; a fibre scope 403 having an image guide consisting of a fibre bunch as an optical viewing means; a mosaic type TV camera 405 removably connected to an eyepeice 404 of this fibre scope 403; a control apparatus 406 containing a light source and video signal processing unit and connected with the electronic endoscope 402, fibre scope 403, and TV camera 405; and a colour CRT monitor 407 as a display means, all connected to this control apparatus 406.

In the electronic endoscope 402, a thick operating base member 412 is connected to the rear end of an elongate insertable part 411 which may be flexible, for example a flexible cable 413 extends sideways from the rear of the operating base member 412, and is provided at the remote tip with a connector 414 provided integrally with an electric system connector 415 and an illuminating system connector 416.

The illuminating system connector 416 is provided with an illuminating system terminal 416a and an air and water feeding system terminal 416b communicating with an air and water feeding channel (not illustrated) provided within the insertable part 411. A frame sequential type electrical system connector socket 417 and an illuminating system connector socket 418 are provided, to which the electric system connector 415 and illuminating system connector 416 are respectively connected, for example on the front face of the control apparatus 406, so that the electronic endoscope 402 may be connected to the control apparatus 406 by these connectors 415 and 416 and connector sockets 417 and 418.

Instead of the field sequential system electronic endoscope 402, a field sequential system TV camera can be connected to the frame sequential type electric system connector socket 417.

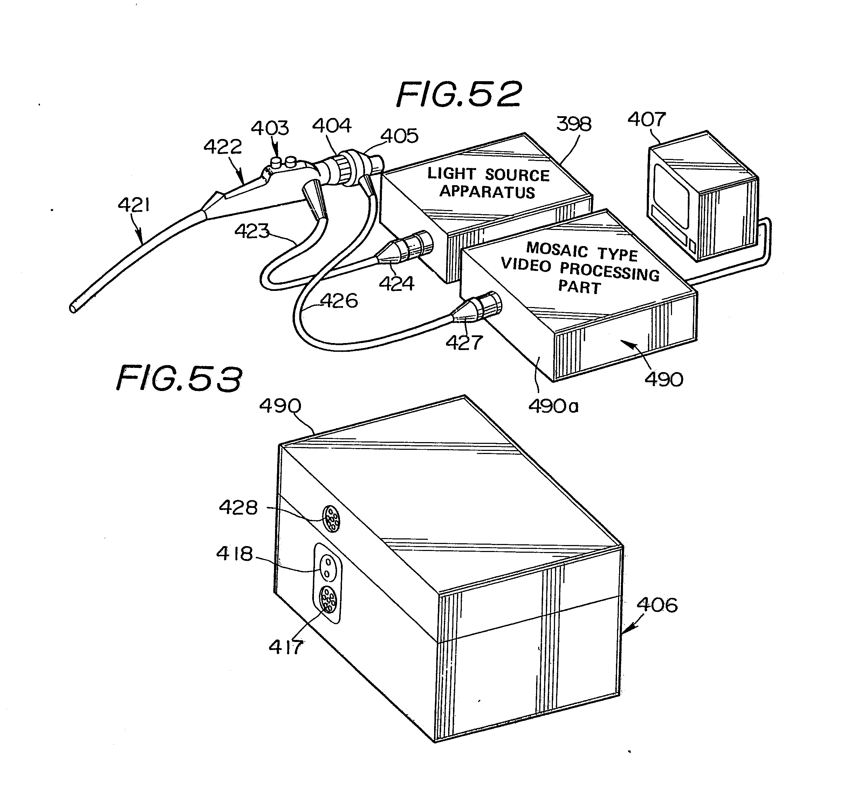

On the other hand, in the fibre scope 403 as in the electronic endoscope 402, a thick operating base member 422 is connected to the rear end of an elongate insertable part 421, preferably a flexible part, for example. A flexible cable 423 extends from the side at the rear end of the operating base member 422, and is terminated by an illuminating system connector 424, provided with an illuminating system terminal 424a and an air and water feeding system terminal 424b communicating with an air and water feeding channel (not illustrated), provided within the insertable part 421. The illuminating system connector 424 is to be connected to the illuminating system connector socket 418. The illuminating system connector 424 can be connected to the illuminating system connector socket 418 of the control apparatus 406, or to various light source apparatus 398 for the endoscopes of the light guide system, as shown in Figure 52.

A flexible cable 426 extends from the side of the TV camera 405, and is terminated by an electric system connector 427. This electric system connector 427 is for connection to a mosaic type electric system connector socket 428 provided adjacent to, but below, the illuminating system connector socket 418 on the front face of the control apparatus 406, for example.

Not only the fibre scope 403 and TV camera 405, but also the mosaic type electronic endoscope having a mosaic type solid-state imaging device at the remote tip of the insertable part can be connected to the illuminating system connector socket 418 and mosaic type electric system connector socket 428.

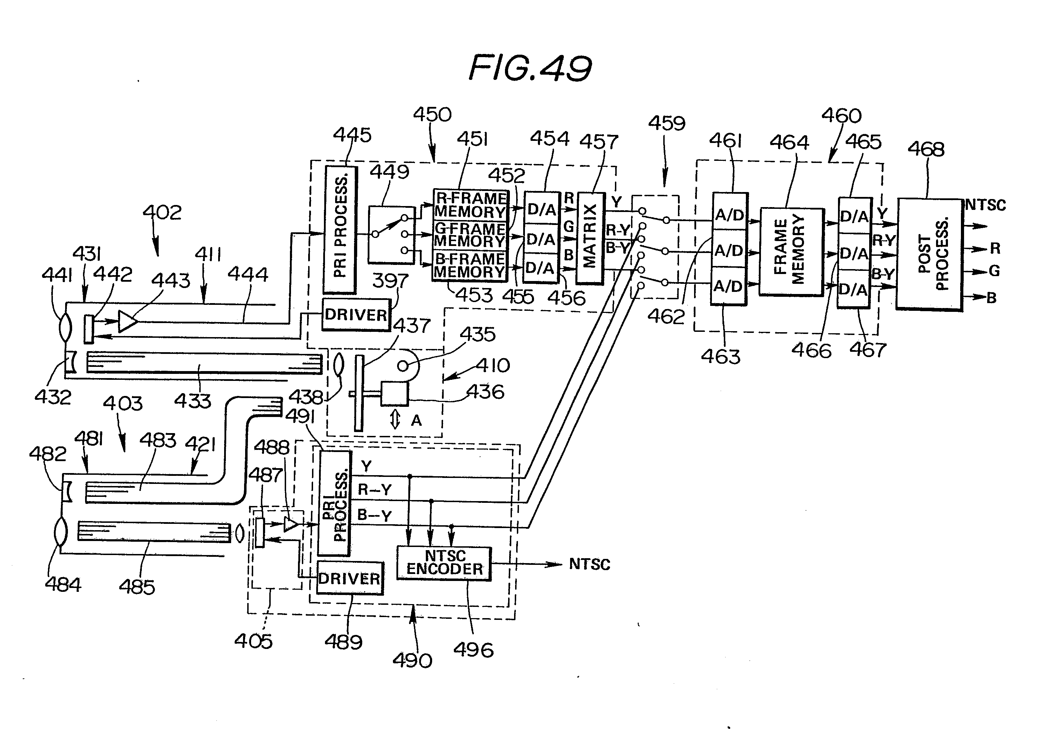

As shown in Figure 49, a light distributing lens 432 is arranged in the distal tip 431 of the insertable part 411 of the elctronic endoscope 432, and the exit face of a light guide 433 consisting of a flexible optical fibre bunch inserted through the insertable part 411 is arranged at the rear of this light distributing lens 432. This light guide 433 is coupled at its entry face at a base end to the illuminating system socket 416a by its connector 414, which is connected to the connector sockets 417 and 418 of the control apparatus 406, the illuminating light emitted from the light source 410 within the control apparatus 406 will be incident upon the light guide 433. The light source 410 is provided with a lamp 435 emitting a white light, and with a rotary colour filter 437 having a triple primary colour transmitting filter, with red (R), green (G) and blue (B) sections, and is rotated by a motor 436. The illuminating light emitted from the lamp 435 is thus made to produce a sequence of lights of the respective wavelengths of red, green and blue in turn, which is condensed by a condenser lens 438 to be incident upon the entry face of the light guide 433.

In this embodiment, the rotary colour filter 437 is movable in the direction indicated by the arrow A to be removably insertable in the path between the lamp 435 and condenser lens 438. The light incident upon the light guide 433 is led to the distal tip 431 by this light guide 433, emitted from the exit face of this light guide 433, and radiated onto an object through the light distributing lens 432.

An image forming optical system 441 consisting of an objective or the like is provided in the tip 431 to return via the electronic endoscope 402. A solid-state imaging device 442 such a CCD is arranged in the image forming plane of this image forming optical system 441. This solid-state imaging device 442 is driven by a frame sequential system driver 397 within the control appartus 406. Returning lights corresponding to the respective colour lights or red, green and blue from the object are received by the solid-state imaging device 442 through the image forming optical system 441. The output signal of this solid-state imaging device 442 is amplified by a pre-amplifier 443 provided within the tip 431 and is fed into a frame sequential system video signal processor stage 450 within the control apparatus 406 through a signal line 444 inserted through a cable 413, and electric system connector 415 of the integrated connector 414, and a frame sequential type electric system connector socket. In this video signal processor stage 450, the output signal of the solid-state imaging device 442 is first input into a pre-processor circuit 445 such as shown, for example, in Figure 50. In this pre-processor circuit 445, a video signal is extracted from the output signal of the solid-state imaging device 442 in a sample holding circuit 446, is $\gamma$-corrected in a $\gamma$-correcting circuit 447, and then converted to a digital signal by an A/D-converter 448. This digital signal is selectively switched in synchronism with the colours of the frame sequential illumination by a multiplexer 449, and is memorized in an R frame memory 451, G frame memory 452 and B frame memory 453, corresponding to the respective colours of red, green and blue in their turn. These frame memories 451,452 and 453 are read out simultaneously at a velocity matching such displaying apparatus as a colour CRT monitor 407, and are converted to respective analogue signals by D/A-converters 454,455 and 456 to produce R,G and B colour signals. These R,G and B colour signals are converted into a luminance signal Y and colour-difference signals R-Y and B-Y by a matrix circuit 457 (Figure 49).

The respective outputs of the luminance signal Y and colour-difference signals R-Y and B-Y from the matrix circuit 457 are connected each to an associated one switching contact of the selector switch 459 and th eluminance signal Y and colour-difference signals R-Y and B-Y are fed into a freezing part 460 through this selector switch 459. In this freezing part 460, the luminance signal Y and colour-difference signals R-Y and B-Y are converted to digital signals respectively by A/D-converters 461,462 and 463, and are then memorized in a frame memory 464. The digital signal read out of the frame memory 464 is converted into analogue form by D/A-converters 465,466 and 467, to be fed into a post-processor circuit 468 as is shown in Figure 51, for example. In this post-processor circuit 468, the luminance signal Y and colour-difference signals R-Y and B-Y are fed into an NTSC encoder 469, converted to an NTSC signal, and fed on as an output signal. The luminance signal Y and colour-difference signals R-Y and B-Y are also fed into an inverse matrix circuit 470, and are converted to R,G and B signals by this inverse matrix circuit 470, from which they are output via respective drivers 471,472 and 473. Thus, in this embodiment, the NTSC signal and R,G and B signals can be output. The NTSC signal, or R,G and B signals are fed into the monitor 407 and an observed image displayed.

In the freezing part 460, at the time of freezing data, one complete frame will be memorized and then the writing into the frame memory 464 will be stopped and a stationary picture image can be displayed by the monitor 407, so that it can be studied.

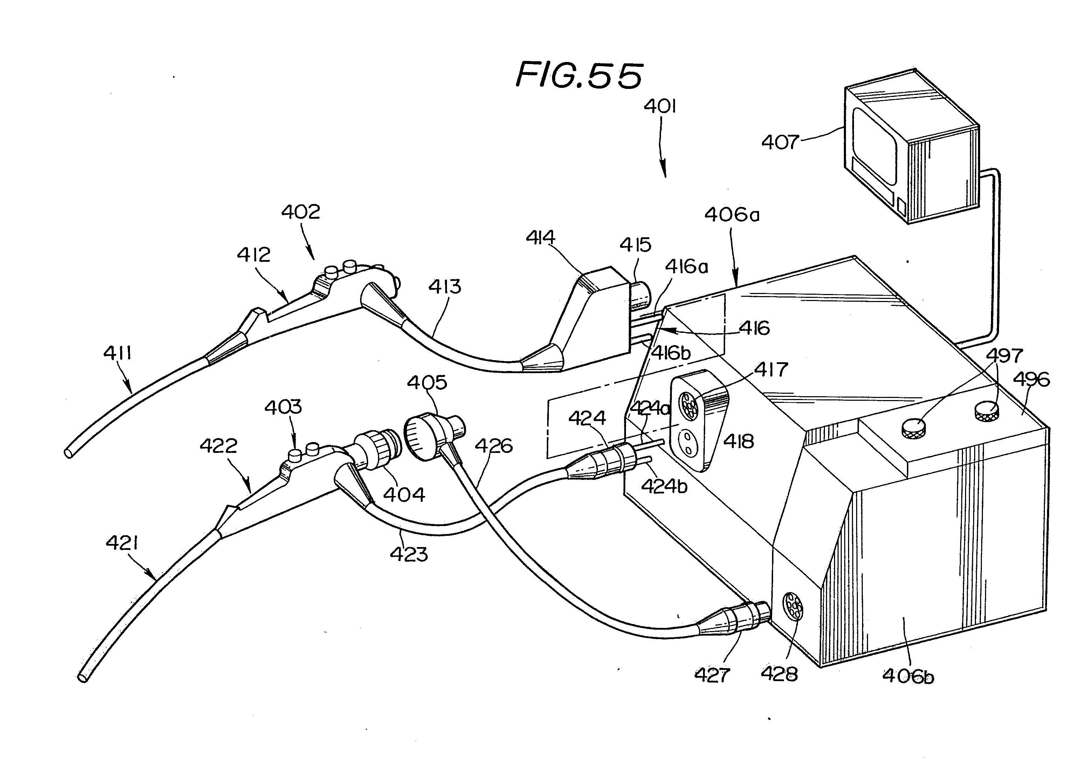

On the other hand, a light distributing lens 482 (Figure 54) is arranged in the distal tip 481 of the insertable part 421 of the fibtre scope 403, and the exit end of a light guide 483 consisting of a flexible optical fibre bunch inserted through the insertable part 421 is arranged behind this light distributing lens 481. This light guide 483 is connected at its base end to the illuminating system connector 424 (Figure 55). The illuminating light emitted from the light source apparatus 410 within the control apparatus 406 is incident upon the light guide 483. In the event of the illuminating system connector 424 of this fibre scope 403 being connected to the illuminating system connector socket 418 of the control apparatus 406, the rotary colour filter 437 of the light source apparatus 410 will be moved from the optical axis in the direction indicated by the arrow A, and white illuminating light emitted from the lamp 435 will be incident upon the light guide 483, without passing through the rotary colour filter 437. The light incident upon the light guide 483 is led by this light guide 483, emitted from the exit end-face of this light guide 483, and radiated onto an object through the light distributing lens 482.

An image forming optical system 484 consisting of an objective or the like is provided in the distal tip 481 of the fibre scope 403. The tip entry-face of an image guide 485 consisting of an optical fibre bunch inserted through the insertable part 421 is arranged in the image plane forming position of this image forming otpical system 484. The observed image formed by the image forming optical system 484 is led to the eyepiece part 404 by the image guide 485 so as to be observed via this eyepeice part 404. The observed image can be relayed out be connecting the TV camera 405 to this eyepeice part 404. The TV camera 405 is provided with a solid-state imaging device 487 arranged in the image forming plane position of the eyepeice part 404, and a pre-amplifier 488 amplfes the output signal of this solid-state imaging device 487. A filter array (not illustrated) in which colour filters transmitting respective colours such as R,G and B is arranged in the form of a mosaic or the like on the front face of the solid-state imaging device 487. The solid-state imaging device 487 is driven by a mosaic type driver 489 within the control apparatus 406. The output signal of the solid-state imaging device 487 is amplified by the pre-amplifier 488 and is fed into a mosaic type video signal processor stage 490 within the control apparatus 406. In this video signal processor stage 490, the output signal of the solid-state imaging device 487 is initially fed into a pre-processor circuit 491 of the same formation as that of the processor circuit 41b shown, for example, in Figure 8.

The luminance signal Y and the colour-difference signals R-Y and B-Y produced by this pre-processor circuit 491 are fed to an encoder 496 to be converted into an NTSC signal output. The respective outputs of the luminance signal Y and colour-difference signals R-Y and B-Y from the pre-processor circuit 491 are also connected to the other switching contacts of the switch 459. The luminance signal Y and colour-difference signals R-Y and B-Y can thus be fed into the freezing part 460 through this selector switch 459. That is to say, in this embodiment the freezing part 460 and post-processor 468 are commonly used by the frame sequential system electronic endoscope 402 and the mosaic type TV camera 405. By switching the selector switch 459 from the post-processor circuit 468, the video signal of the observed image by the electronic endoscope 402, and the video signal of the observed image produced by the fibre scope 403 and TV camera are selectively switched through to the output.

In this embodiment, as shown in Figures 48 and 52, the mosaic type video signal processor 490 is contained within a housing 490a, made up as a unit removably fitted to the control apparatus 406, and can be used with only the mosaic type imaging means. This unit video signal processor 490 can be removably inserted into the control apparatus 406 from the front face. When it is fltted to the control apparatus 406, the respective outputs of the luminance signal Y and colour-difference signals R-Y and B-Y of the pre-processor circuit 491 will be connected to the switching contacts of the selector switch 459. Also, the mosaic type electric system connector socket 428 is provided on the front face of the housing 490a (Figure 48) of the mosaic system video signal processor.

Therefore, as shown in Figure 52, when the illuminating system connector 242 of the fibre scope 403 is connected to the light source apparatus 398 for the light guide system endoscope, and the electric system connector 427 of the TV camera 405 connected to the eyepiece part 404 of this fibre scope 403 is connected to the electric system connector socket 428 of the video signal processor 490, an NTSC signal will be available from the video signal processor 490 and the observed image by the mosaic system can be displayed on the monitor 407.

Thus, in this embodiment, there is provided the frame sequential system electronic endoscope 402; fibre scope 403 and mosaic type TV camera 405 connected to the eyepiece part 404 of this fibre scope 403; and the control apparatus 406 to which they are connected. Within the control apparatus 406, there is provided the light source apparatus 410 which can feed a frame sequential illuminating light adapted to the electronic endoscope 402 and a white illuminating light adapted to the TV camera 405; frame sequential system video signal processor 450; and mosaic type video signal processor 490. Therefore, both the electronic endoscope 402 (which is a frame sequential system imaging means), and the TV camera 405 connected to the fibre scope 403 (which is a mosaic type imaging means), can be used.

Further, the mosaic type video signal processor 490 is made of a unit removably fitted to the control apparatus 406 and can be used with only the mosaic type imaging means. Therefore the TV camera 405 connected to the fibre scope 403, which is a mosaic type imaging means, can be used alone.

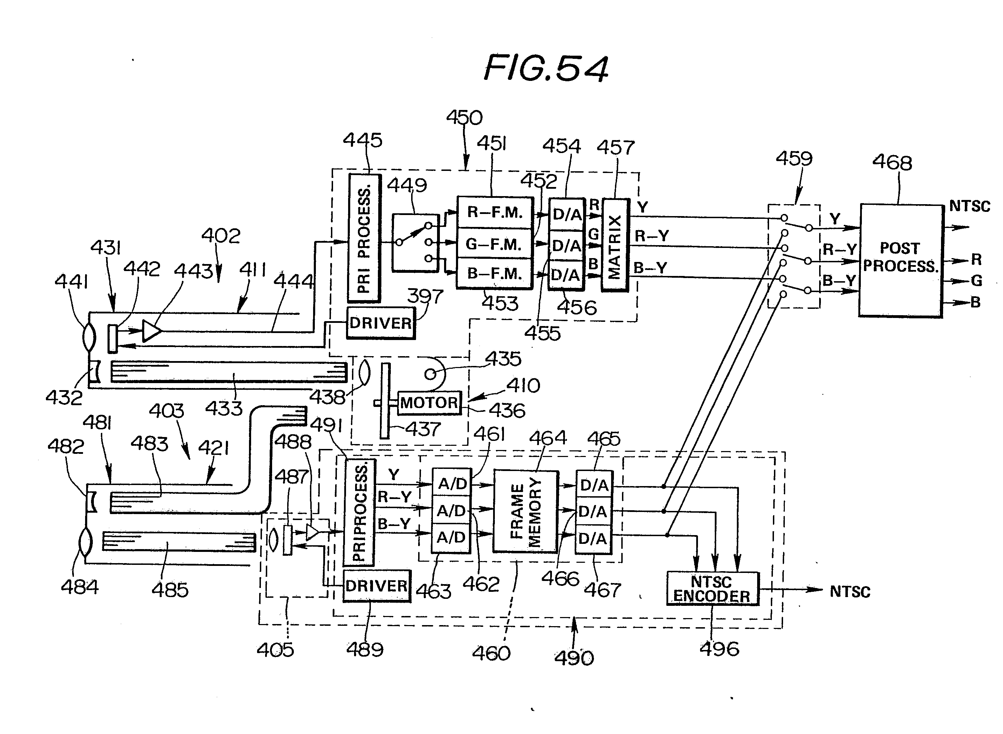

Figure 53 is a perspective view of a control apparatus relating to the fifteenth embodiment of the present invention.

In this embodiment, the mosaic type video signal processor stage 490 can be removably fitted on the upper

17

face of the other part of the control apparatus 406. When it is mounted on the upper face of the other part of the control apparatus 406, the respective outputs of the luminance signal Y and the colour-difference signals R-Y and B-Y of the pre-processor circuit 491 will be connected to the switching contacts of the selector switch 459.

The other elements, operations and functions are the same as those in the fourteenth embodiment.

Figure 54 is a block diagram showing the formation of an endoscope system relating to the sixteenth embodiment of the present invention.

In this embodiment, an image freezing stage 460 within the control apparatus 406 is not provided between the selector switch 459 and post-processor 468, but is provided within the mosaic type video signal processor stage 490. The luminance signal Y and colour-difference signals R-Y and B-Y are fed to the NTSC encoder 499 and the selector switch 459 through the frame memory store 464 of the image freezing stage 460. The other formations are the same as in the fourteenth embodiment.

According to this embodiment, even in the event that the TV camera 405 is connected to the fibre scope 403 which is a mosaic type imaging means being used alone. by using the freezing stage 460, the NTSC signal can be frozen, and a stationary picture image can be displayed.

If a frame sequential type imaging means is used, a stationary picture iamge can be displayed by using the frame memories 451,452 and 453 within the frame sequential type video signal processor stage 450.

The other operations and effects are the same as those in the fourteenth embodiment.

The present invention is not limited to the embodiment described above. For example, the frame sequential system imaging means may be the frame sequential type TV camera connected to the eyepiece part 404 of the optical fibre scope 403, or the mosaic type imaging means may be the electronic endoscope provided with the mosaic type solid-state imaging device in the tip of the insertable part.

In case the frame sequential type and mosaic type commonly use the light source apparatus 410, the connector sockets may be separately provided to move the lamp. The rotary colour filter 437 may be provided with a transparent part so that, when the mosaic type imaging means is used, white light is emitted from the lamp 435 to be incident upon the light guide 483 through this transparent part. Also, the light source apparatus for the frame sequential type and for the mosaic type may be separately provided.

The electric system connector socket and illuminating system connector socket may be respectively common or separate.

Further, the frame sequential type video signal processor stage 450, or both of the frame sequential type video signal processor stage 450 and mosaic type video signal processor stage 440, may be removably fitted to the control apparatus 406. The removably fitting means is not limited to the ones shown in the fourteenth and fifteenth embodiments. There can be used such various means as, for example, removably connecting both video signal processor stages 450 and 490 on the right and left.

The frame sequential system and mosaic system video signal processor circuits are not limited to be partly used in common, as in the embodiment just described, but may be separately provided.

Further, not only the monitor 407 but also a picture image file monitor type still camera and/or video tape recorder may be connected to the control apparatus 406.

In the system of the seventeenth embodiment of the present invention shown in Figure 55, the mosaic type control apparatus unit 406b is to be fitted to the side of the frame sequential type control apparatus 406a.

In this system, the mosaic type video processor stage 490 is made fittable to the side of the control apparatus 406 in the system 401 shown in Figure 48. The structure of the fitting part is shown in Figure 56.

In this embodiment, as shown in Figures 55 and 56, a housing 550 containing the frame sequential type video signal processor stage 450a is provided with a lid 596 on one side face so as to be opened upward by hinges 595 which are opening and closing members. Further, as in Figure 55, the frame sequential type housing 550a and the mosaic type housing 590a are removably fitted by screwing fixing screws 597 as jointing means provided rotatably on the lid 596 so as not to drop off into female screw-parts provided on the upper surface of the housing 590a containing the mosaic type video signal processor stage 490.

A connector socket 575 electrically connecting the frame sequential type control apparatus 406a and mosaic type control apparatus 406b is provided on the side 594 to which the frame sequential type housing 550a and mosaic type housing 590 are applied. A connector socket 576 is provided on the side plate of the mosaic type housing 590a corresponding to the connector socket 575. By doing this, the mosaic type housing 590a can be coupled to the frame sequential type housing 550 and, at the same time, the circuits can be connected.

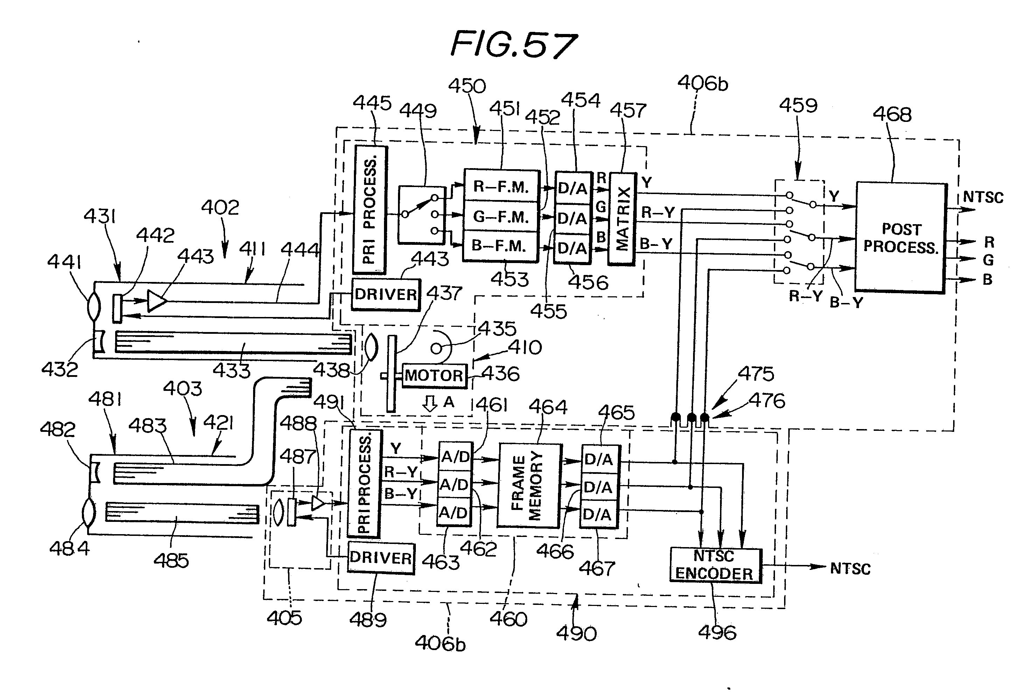

As shown in Figure 57, the formation of the electric system of the system of this embodiment is substantially the same as that shown in Figure 49.

According to this embodiment, the housings 550a and 590a can be fitted simply and positively. In the case of using only the frame sequential type control apparatus 406a, if the lid 496 is made to fall on the side 494, it will not be in the way and, even in case the mosaic type control apparatus 406b is coupled, the unit fitting structue has a good appearance.

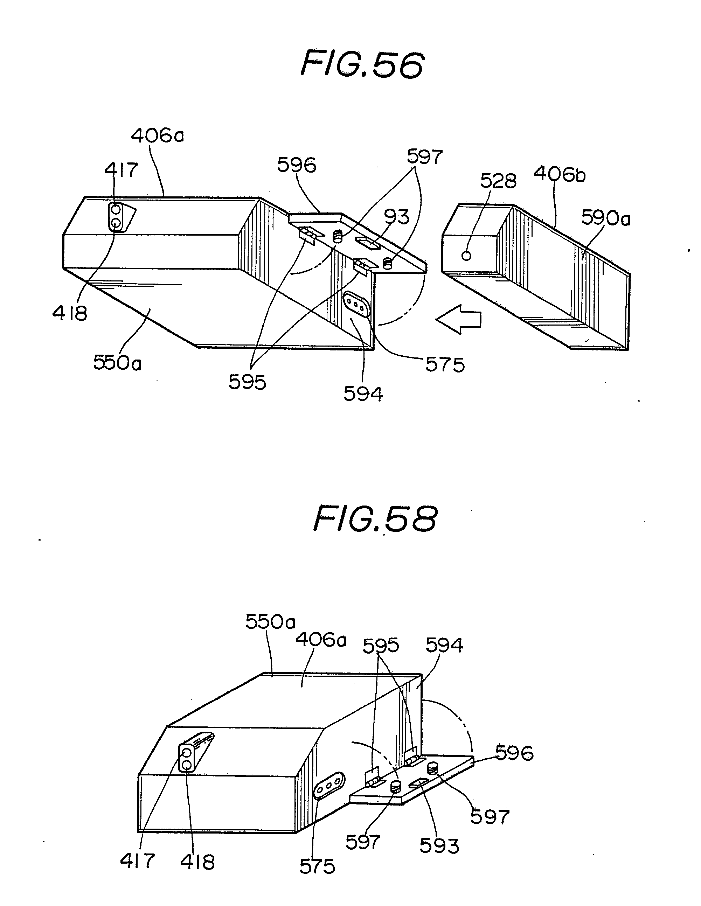

Figure 58 is a perspective view of a formation showing a coupling means of a frame sequential type control apparatus relating to a modification of the seventeenth embodiment of the present invention.

In this modification, the housing 550a containing the frame sequential type video signal processor stage 450 is provided on one side with a lid 596 so as to be opened downward by hinges 595 which are opening and closing members. Further, the frame sequential type housing 550a and the mosaic type housing 590a are removably fitted by screwing fixing screws 597 as fixing means provided rotatably on the lid 596 so as not to drop off into female screw-parts provided on the lower surface of the housing 590a containing the mosaic type video signal processor stage 490. The lid 596 is provided with a magnet 593 which can attract the side wall 594 of the housing 550a made, for example, of a ferrous metal so that, in case the mosaic type control apparatus 406b is not fitted, the lid 96 may be held securely against the side 594.

The other formations are the same as in the seventeenth embodiment.

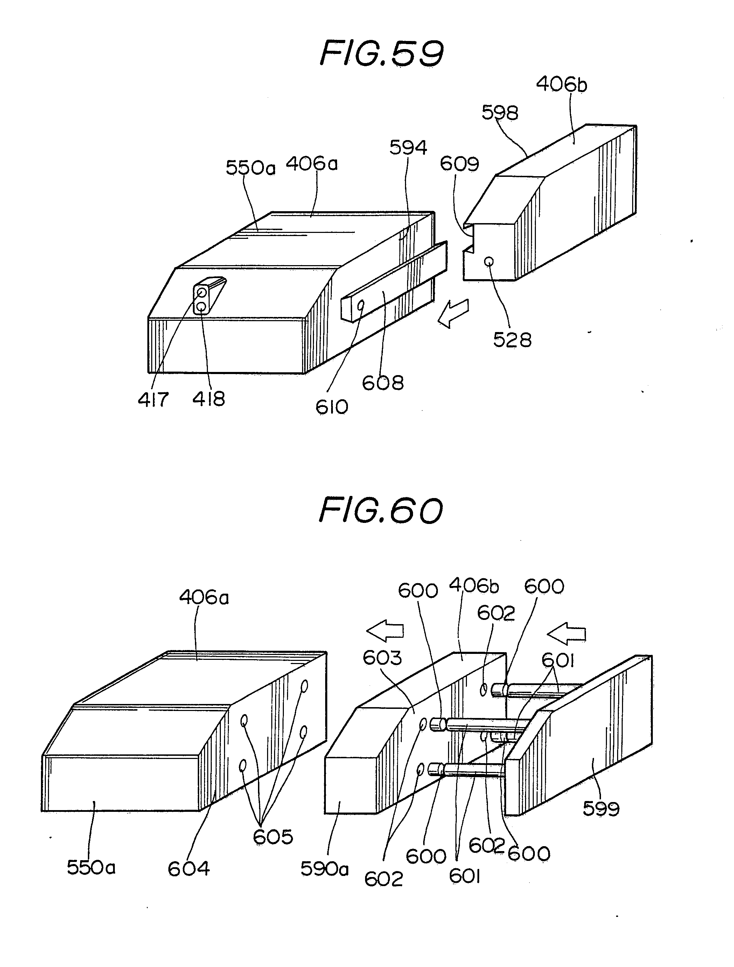

Figure 59 is a perspective view of a formation showing a coupling means of a frame sequential system control apparatus relating to the eighteenth embodiment of the present invention.

In this embodiment, a guide projection 608 expanding to its outer face is provided on the side face 594 from the rear of the frame sequential type control apparatus 406a contained in the housing 550a, and a recess 509 (mating with the guide projection) is provided on the side 598 of the housing of the mosaic type control apparatus 406b corresponding to the frame sequential type control apparatus, so that the frame sequential type control apparatus 406a and mosaic type control apparatus 406b may be fitted and coupled to each other.

A click-stop device 610 is provided on the face of the guide projection 608 so that the frame sequential type control apparatus 406a and mosaic type control apparatus 406b may be coupled in a predetermined position and may further be electrically connected at the correct coupling point.

The other formations are the same as in the seventeenth embodiment.

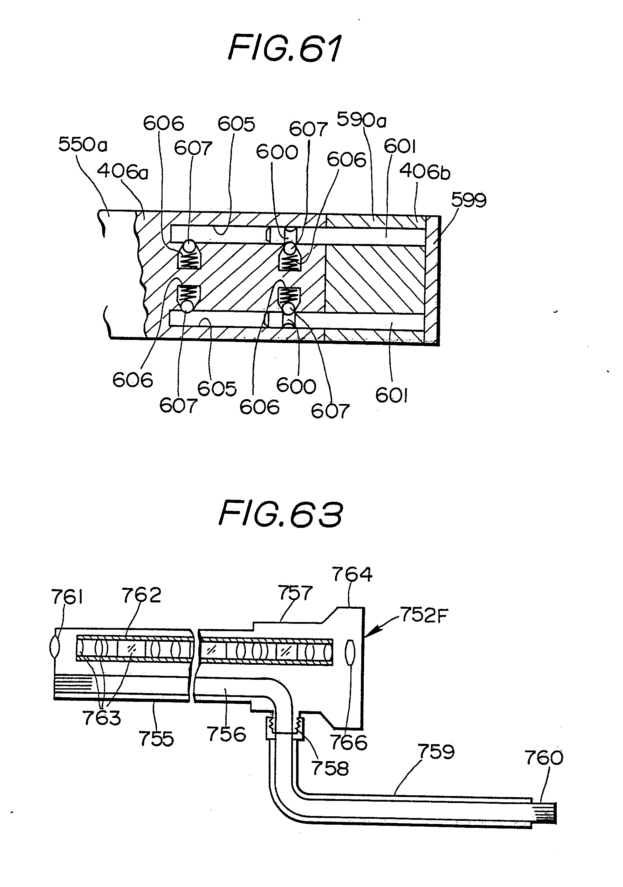

Figure 60 relates to the nineteenth embodiment of the present invention, and shows a coupling means of the frame sequential type control apparatus and mosaic type control apparatus. Bars 601 each having a peripheral groove 600 near the tip are provided extending from one side of a lid 599 of the same shape as of the sides 603 and 604 of the frame sequential type and mosaic type housings 550a and 590a. Through holes 602 and blind holes 605 are provided respectively on the side 603 of the housing 590a of the mosaic type control apparatus 406b corresponding to the bars 601, and on the side 604 of the housing 550a of the same sequential type control apparatus 406a. Balls 607 spring loaded by springs 606 are provided respectively in the position reached by the groove 600 of the bar 601 when the mosaic type housing 590a is inserted (as shown in detail in Figure 61) to engage in the blindhole 605, and in the position reached by the groove 600 of the bar 601 when the mosaic type housing 590a is not inserted. The lid 599 is jointed to the housing 550a, and a second set for the position reached when the housing 590a is in place, as illustrated. When the bar 601 is inserted in the blind hole 605, the bar 601 will enter the hole against the energising force of the spring 606. When the ball 607 reaches the position of the groove 600, the ball 607 will fit in the groove 600 and the lid 599 will be fixed.

The other formations are the same as in the seventeenth embodiment.

In the system of each of the above-described embodiments, a fibre scope is used as an optical endoscope but a rigid endoscope can also be used.

For example, in the system 1 of the first embodiment, not only the fibre scope 2E, and the fibre scopes 2C and 2D fitted with TV cameras, but also the rigid endoscope 752F shown in Figure 2, the rigid endoscope 752G fitted with the frame sequential TV camera 753 or mosaic type TV camera 754 in the rigid endoscope 752F, and the rigid endoscope 752H fitted with the mosaic type TV camera, can be used.

In the rigid endoscope 752F, as shown in Figure 63, a light guide 756 is inserted through a rigid insertable part 755 and is fixed at the end on the base side with a light guide mouthpiece 758 of a holding part 757. A light guide cable 759 can be connected to this light guide mouthpiece 758; has a light guide connector 760 formed; transmits the illuminating light fed from the light source to the light guide 756; and emits it from the exit end-face at the distal tip of the insertable part 755. The object illuminated by the illuminating light emitted from this exit end-face is made to form an image in the focal plane by an objective 761 fitted to the tip. The optical image formed by this objective is trasmitted to the exit end-face adjacent the eyepiece 164 by a relay optical system 763 fitted through a rigid lens tube 762, and can be observed with a naked eye through an eyepiece lens arranged within an eyepiece 764. When a removable TV camera 753 or 754 is fitted to this eyepiece 764, the image can be formed in the imaging plane of a CCD 767 by an image forming lens 766. A colour separating mosaic filter 768 is fitted to the imaging surface of the CCD 767 in the mosaic type TV camera 754.

Signal cables 769 are connected from the TV camera 753 and 754, and are fitted at the ends with respective signal connectors 770G and 770H.

The TV cameras 753 and 754 contain respective type signal generating circuits 771G and 771H (abbreviated as T.S.G.) which emit type signals respectively indicating the frame sequential type and mosaic type colour imaging means.

The rigid endoscope 752F or rigid endoscopes 752G and 752H fitted respectively with the frame sequential type TV cameras 753 and 754 can be applied also to the other embodiments by changing the connector 760 or 770G and 770H.

In the embodiments described, there can be provided a correcting circuit means for correcting the temperature dependency of the ligh emitting characteristics of the light source lamp 31 or the like.

Different embodiments can be formed by combining parts of the respective embodiments and these also belong to the present invention.

As described above, according to the present invention, a signal processor for processing mutually-different signals corresponding respectively to colour imaging scopes or different types and systems is formed to display the imaes by a colour monitor, and therefore scopes of different types can be used at will as desired, and can be changed without having to replace the imaging apparatus itself.

## Claims

1. An endoscope imaging system comprising:
a colour imaging means provided with a colour filter using a solid-state imaging device fitted with a colour separating filter in front of an imaging part having a photo-electric transducer function;
a frame sequential type colour imaging means using a solid-state imaging device fitted with a colour separating filter in front of an imaging part having a photo-electric transducer function;
light output means and signal processing means, either or both of which is provided with characteristics corresponding to said colour imaging means provided with said colour filter and said frame sequential type colour imaging means; and
a colour monitor displaying pre-selected video signals from said signal processing means.

2. An endoscope imaging system comprising:
a colour imaging means provided with a colour filter using a solid-state imaging device fitted with a colour separating filter in front of an imaging part having a photo-electric transducer function;
light output means emitting a white light having components corresponding to characterics of said colour imaging means provided with said colour filter;
a signal processing means provided with the requisite characteristics for processing the signal corresponding to said colour imaging means provided with said colour filter, and for processing the signal corresponding to said frame sequential type colour imaging means; and
a colour monitor displaying pre-selected video signals from said signal processing means.

3. An endoscope imaging system comprising:
a colour imaging means provided with a colour filter using a solid-state imaging devide fitted with a colour separating filter in front of an imaging part having a photo-electric transducer function;
light output means emitting a white light with colour components corresponding to those of said coloud imaging means provided with said colour filter, and emitting a frame sequential light sequence in mutually-different wavelength sections;
a signal processing means processing the signal corresponding to said colour imaging means provided with said colour filter; and
a colour monitor displaying pre-selected video signals from said signal processing means.

4. An endoscope imaging system comprising:
a frame sequential type colour imaging means using a solid-state imaging device not fitted with a colour filter in front of an imaging part having a photo-electric transducer function;
a light output mans emitting frame sequential light pulses in mutually-different wavelength ranges;
a signal processing means provided with the characteristics required for processing the signal corresponding to said frame sequential type colour imaging means, and for processing the signal corresponding to said colour imaging means provided with said colour filter; and
a colour monitor displaying pre-selected video signals from said signal processing means.

5. An endoscope imaging system comprising:
a frame sequential type colour imaging means using a solid-state imaging device not fitted with a colour filter in front of an imaging part having a photo-electric transducer function;
a light output means emitting frame sequential light pulses in mutually-different wavelength ranges, and emitting a white light;
a signal processing means processing the signal corresponding to said frame sequential type colour imaging means; and
a colour monitor displaying pre-selected video signals from said signal processing means.

6. An endoscope imaging system as claimed in Claim 2, characterised in that said light output means has a function of emitting frame sequential light pulses in mutually-different wavelength ranges.

7. An endoscope imaging system as claimed in Claim 4, characterised in that said light output means has a function of emitting a white light.

8. An endoscope imaging system as claimed in Claim 1, characterised in that said light output means is provided with both the function of a white light output means, and a frame sequential type light pulse output emitting illuminating light pulses in mutually-different wavelength ranges.

9. An endoscope imaging system as claimed in Claim 1, characterised in that said signal processing means has a function of processing the signal corresponding to said colour imaging means provided with said colour filter and a function of processing the signal corresponding to said frame sequential type colour imaging means.

10. An endoscope imaging system as claimed in any one of Claims 1 to 3, characterised in that said colour imaging means provided with a colour filter is contained in the distal tip of an elongate insertable part of an electronic scope.

11. An endoscope imaging system as claimed in any one of Claims 1, 4 and 5, characterised in that said frame sequential type colour imaging means is contained in the distal tip of an elongate insertable part of an electronic scope.

12. An endoscope imaging system as claimed in any one of Claims 1 to 3, characterised in that said colour imaging means provided with said colour filter is contained within a TV camera fittable to an eyepeice part of an optical endoscope having an image guide.

13. An endoscope imaging system as claimed in any one of Claims 1, 4 and 5, characterised in tht said frame sequential type colour imaging means is contained within a TV camera fittable to an eyepiece part of an optical endoscope having an image guide.

14. An endoscope imaging system as claimed in Claim 12, characterised in that said optical endoscope is a fibre scope in which said image guide is formed of an optical fibre bunch.

15. An endoscope imaging system as claimed in Claim 12, characterised in that said optical endoscope is a rigid endoscope in which said image guide is formed by an optical relay system.

16. An endoscope imaging system as claimed in Claim 13, characterised in that said optical endoscope is a fibre scope in which said image guide is formed by an optical fibre bunch.

17. An endoscope imaging system as claimed in Claim 13, characterised in that said optical endoscope is a rigid endoscope in which said image guide is formed by an optical relay system.

18. An endoscope imaging system as claimed in Claim 6, further comprising a frame sequential type colour imaging means.

19. An endoscope imaging system as claimed in Claim 7, further comprising a colour imaging means provided with a colour filter.

20. An endoscope imaging system as claimed in any one of Claims 1 to 5, characterised in that said light output means and said signal processing means are contained within a common housing.

21. An endoscope imaging system as claimed in any one of Claims 1 to 5, characterised in that said light output means and said signal processing means are respectively contained within separate housings.

22. An endoscope imaging system as claimed in any one of Claims 2, 4 and 9, characterised in that said signal processing means has a common signal connector socket connectable with signal connectors fitted respectively to said colour imaging means provided with said colour filter and said frame sequential type colour imaging means, and said light output means has a single light source connector socket.

23. An endoscope imaging system as claimed in any one of Claims 3, 5, 6 and 7, characterised in that said light output means has a common light source connector socket selectively emitting said frame sequential light pulses and said white light, and said signal processing means has a single signal connector socket.

24. An endoscope imaging system as claimed in Claim 18 or 19, characterised in that said colour imaging means provided with said colour filter, and said frame sequential colour imaging means have type signal generating means emitting respective, mutually-different type signals.

25. An endoscope imaging system as claimed in Claim 24, characterised in that said light output means has a switching means capable of selectively emitting said frame sequential light pulses or said white light.

26. An endoscope imaging system as claimed in Claim 25, characterised in that said signal processing means has a detector means for discriminating said type signal.

27. An endoscope imaging system as claimed in Claim 26, characterised in that said switching means is controlled by the discriminating signal from said type signal discriminating means.

28. An endoscope imaging system as claimed in any one of Claims 2, 4 and 9, characterised in that said signal processing means is formed of a first signal processing stage for processing the signal corresponding to said colour imaging means provided with said colour filter, and a second, different signal processing stage for processing the signal corresponding to said frame sequential type colour imaging means.

29. An endoscope imaging system as claimed in Claim 24, characterised in that said signal processing means is formed of a first signal processing stage for processing the signal corresponding to said colour imaging means provided with said colour filter, and a second, different signal processing stage for processing the signal corresponding to said frame sequential type colour imaging means.

30. An endoscope imaging systemas claimed in Claim 29, characterised in that said signal processing means has said type signal discrimination means.

31. An endoscope imaging system as claimed in Claim 30, further comprising switch means selectively switching between said first signalprocessing stage and said second signal processing stage.

32. An endoscope imaging system as claimed in Claim 30, characterised in that said discriminating means discriminates said type signals and switches said switching means.

33. An endoscope imaging system as claimed in Claim 28, characterised in that, of said first signal processing stage and said second, different signal processing stage, one signal processing stage can be physically fitted to the other signal processing stage.

34. An endoscope imaging system as claimed in Claim 28, characterised in that said first signal processing stage and said second, different signal processing stage are contained in a common housing.

35. An endoscope imaging system as claimed in Claim 28, characterised in that said first signal processing stage and said second, different signal processing stage have a common circuit utilising a common section of the imaging system circuit.

36. An endoscope imaging system as claimed in Claim 34, characterised in that said first processing stage and said second, different signal processing stage have at least the output stage in common.

37. An endoscope imaging system as claimed in Claim 34 or 35, characterised in that said first signal

**0 277 792**

processing stage and said second, different signal processng stage produce NTSC system colour video signals.

38. An endoscope imaging system as claimed in Claim 35, <u>characterised in that</u> said common circuit has a frame freezing memory.

39. An endoscope imaging system as claimed in Claim 33, <u>characterised in that</u> a first housing containing said first signal processing stage can be fitted to a fitting port provided on a second housing containing said second, different signal processing stage.

40. An endoscope imaging system as claimed in Claim 33, <u>characterised in that</u> said first housing containing said first signal processing stage can be fitted to the side of said second housing containing said second, different signal processing stage.

41. An endoscope imaging system as claimed in Claim 39, <u>characterised in that</u> said fitting port is provided on the front face of said second housing.

42. An endoscope imaging system as claimed in Claim 39, <u>characterised in that</u> said fitting port is provided on the back face of said second housing.

0277792

# FIG.1

I ENDOSCOPIC IMAGING SYSTEM

333 IMAGING APPARATUS

11a

13 COLOR MONITOR

2A ELECTRONIC SCOPE OF FRAME SEQUENTIAL TYPE

5A

6A

4

3

12a    12b    11b

6C

8C    5C

2B ELECTRONIC SCOPE WITH MOSAIC FILTER

5B

4

6B

4

3

2C FIBER SCOPE ATTACHED T.V. CAMERA OF FRAME SEQUENTIAL TYPE

3

2D FIBER SCOPE ATTACHED T.V. CAMERA WITH MOSAIC FILTER

6D

2E FIBER SCOPE    7    5E

8D

4

5D

3    4    3

0277792

FIG.2

0277792

**FIG.3**

**FIG.4**

**FIG.5**

## FIG.6

## FIG.7

0277792

## FIG.8

## FIG.10

0277792

# FIG.9
## (a)

## (b)

0277792

## FIG. 11

LIGHT EMIT. DEVICE — 94

91

92

93

LIGHT RECEIV. DEVICE — 95

DETECTING CIRCUIT — 96

## FIG. 13

2B

73B

2D

74'

75B'

2E

74'

71b'

72'

71a'

101

73A'

2A

75A

74'

75A

2C

0277792

# FIG.12

0277792

## FIG.14

## FIG.15

0277792

# FIG.16

# FIG.17

# FIG.18

FIG.19

0277792

POWER CIRCUIT

TIMING GEN. 52a

MOVEMENT CONTR. C.C. 135

FRAME SEQ. PROCESS. C.C. 41a

R
G
B

DRIVER 26a

DISCR. C.C. 28a

ALARM. C.C. 66a

MOSAIC PROCESS. C.C. 41b

Y
R—Y
B—Y

DRIVER 26b

DISCR. C.C. 28b

ALARM. C.C. 66b

TIMING GEN. 52b

TYPE SIG. GEN. 27A

MOTOR 32a

143
142
71b
14
2A
34
31
33a 51a
12b
141
46
43
113

0277792

## FIG.20

## FIG.22

0277792

# FIG.21

## FIG.23

## FIG.24

0277792

## FIG.25

## FIG.26

FIG.27

0277792

## FIG.28

## FIG.29

0277792

# FIG.30

0277792

## FIG.31

0277792

# FIG.32

0277792

## FIG.33

## FIG.37

0277792

# FIG.34

FIG.35

# FIG.36

0277792

# FIG.38

# FIG.39

0277792

## FIG.40

13 COLOR MONITOR

254

253

252a IMAGING AP.

252b MOSAIC TYPE PREPROCESSOR UNIT

## FIG.41

13

71

254

72

252a

FIG.42

0277792

## FIG.43

284

252b UNIT

253

252a
IMAGING AP.

282  281  283

## FIG.44

284  282 281

282

284

## FIG.45

292a IMAGING AP.

292b UNIT

## FIG.46

292a IMAGING AP.

293

292b
UNIT

0277792

## FIG.47

302a IMAGING AP.

303

302b UNIT

## FIG.50

CCD 442 443 446 447 445 448 449 451 R-F.M. 454 D/A R

452 G-F.M. 455 D/A G

453 B-F.M. 456 D/A B

S/H γ A/D

## FIG.51

468 469 NTSC ENCODER NTSC

470 INVERSE MATRIX

Y

R-Y

B-Y

471 R

472 G

473 B

# FIG.48

401

402
412
413
414
415
406
407
411
416a
416
416b
417
403
405
426
424a
418
422
404
424
428
421
423
427
424b
490a
490

0277792

FIG.49

FIG.52

LIGHT SOURCE
APPARATUS

398

407

422  403  404  405

421

423

426  424

427

MOSAIC TYPE
VIDEO PROCESSING
PART

490

490a

FIG.53

490

428

418

417

406

FIG.54

FIG.55

FIG.57

0277792

## FIG.56

## FIG.58

0277792

# FIG.59

# FIG.60

0277792

## FIG.61

## FIG.63

# FIG.62